# EUROPEAN PATENT SPECIFICATION

(11) **EP 2 177 896 B1**
(45) Date of publication and mention of the grant of the patent: **02.05.2018**
(21) Application number: 08790341.5
(22) Date of filing: 01.08.2008
(51) Int. Cl.: G01N 21/17, A61B 3/12, A61B 3/10, G01N 21/47

(54) **OPTICAL IMAGE MEASURING DEVICE**
OPTISCHE BILDMESSUNGSVORRICHTUNG
DISPOSITIF DE MESURE D'IMAGE OPTIQUE

(30) Priority: 13.08.2007 JP 2007210636
(43) Date of publication of application: 21.04.2010
(73) Proprietor: Kabushiki Kaisha TOPCON, Tokyo 174-8580 (JP)
(72) Inventor: HAYASHI, Takefumi, Tokyo 174-8580 (JP); YUMIKAKE, Kazuhiko, Tokyo 174-8580 (JP); TSUKADA, Hisashi, Tokyo 174-8580 (JP)
(74) Representative: Gagel, Roland
(86) International application number: PCT/JP2008/002077
(87) International publication number: WO 2009/022452

(56) References cited:
- WO-A2-2006/039091
- WO-A2-2007/084750
- JP-A- H10 267 631
- JP-A- 2004 528 111
- JP-A- 2005 265 440
- JP-A- 2005 283 471
- JP-A- 2005 283 472
- US-A1- 2005 018 133
- US-A1- 2007 127 033

## Description

### TECHNICAL FIELD

The present invention relates to an optical image measurement device that radiates a light beam to a measured object and detects an interference light based on the reflected light to thereby form an image of the measured object. The invention is set out in the appended claims that follow.

### BACKGROUND ART

In recent years, such an optical image measurement technique has received attention that forms an image representing the surface morphology or internal morphology of a measured object by using a light beam from a laser light source or the like. This optical image measurement technique does not have invasiveness to a human body unlike an X-ray CT device, and therefore, is expected to be applied particularly in the medical field.

Patent Document 1 discloses an optical image measurement device having such a configuration that: a measuring arm scans an object by using a rotary deflection mirror (a Galvano mirror); a reference mirror is disposed to a reference arm; at the outlet thereof, such an interferometer is used that the intensity of a light appearing due to interference of light fluxes from the measuring arm and the reference arm is analyzed by a spectrometer; and the reference arm is provided with a device that gradually changes the light flux phase of the reference light in non-continuous values.

The optical image measurement device disclosed in Patent Document 1 uses a method of the so-called "Fourier Domain OCT (Optical Coherence Tomography)." That is to say, the morphology in the depth direction (the z-direction) of a measured object is imaged by radiating a low-coherence light beam to the measured object, acquiring the spectrum intensity distribution of the reflected light, and subjecting the acquired distribution to Fourier transform.

Furthermore, the optical image measurement device described in Patent Document 1 is provided with a Galvano mirror that scans with a light beam (a signal light), thereby being capable of forming an image of a desired measurement target region of a measured object. Because this optical image measurement device scans with the light beam only in one direction (the x-direction) orthogonal to the z-direction, a formed image is a two-dimensional tomographic image in the depth direction (the z-direction) along a scan direction of the light beam (the x-direction).

Patent Document 2 discloses a technique of scanning with a signal light in both the horizontal direction and the vertical direction to thereby form a plurality of two-dimensional tomographic images in the horizontal direction and, based on the plurality of tomographic images, acquiring and imaging three-dimensional tomographic information of a measurement range. As a method for three-dimensional imaging, for example, a method of arranging and displaying a plurality of tomographic images in the vertical direction (referred to as stack data or the like), and a method of forming a three-dimensional image by subjecting a plurality of tomographic images to a rendering process can be employed.

Patent Document 3 discloses a configuration in which such an optical image measurement device is applied in the ophthalmic field.

Patent Documents 4 and 5 disclose other types of optical image measurement devices. Patent document 4 describes such a type of optical image measurement device that changes a wavelength of a light radiated to a measured object. This optical image measurement device is called the Swept Source type or the like.

Further, Patent Document 5 describes an optical image measurement device that radiates a light having a predetermined beam diameter to a measured object to form an image of a cross section orthogonal to the traveling direction of the light. This optical image measurement device is called the full-field type, en-face type or the like.

Patent Document 6 describes a Fourier-domain optical coherence tomography (OCT) imager according to the preamble of claim 1, and having an auto-alignment process. The auto-alignment process automatically adjusts at least one optical component of a spectrometer of the imager so that the spectrometer is aligned during an imaging session.

[Patent Document 1] Japanese Unexamined Patent Application Publication No. 11-325849
[Patent Document 2] Japanese Unexamined Patent Application Publication No. 2002-139421
[Patent Document 3] Japanese Unexamined Patent Application Publication No. 2003-543
[Patent Document 4] Japanese Unexamined Patent Application Publication No. 2007-24677
[Patent Document 5] Japanese Unexamined Patent Application Publication No. 2006-153838
[Patent Document 6] WO 2007/084750 A2

### DISCLOSURE OF THE INVENTION

### PROBLEM THAT THE INVENTION IS TO SOLVE

When executing a measurement by an optical image measurement device, there is a case that the intensity of an interference light decreases due to the condition of a measured object or the like and a clear image cannot be obtained.

For example, in a case that the optical media of an eye is opaque due to cataract or the like, the intensity of a signal light propagated through the eye decreases. Consequently, there is a case that the intensity of an interference light decreases and a clear image of the fundus oculi cannot be obtained.

The present invention was made for solving such a problem, and an object of the present invention is to provide an optical image measurement device capable of acquiring a clear image even when the intensity of an interference light is low.

### MEANS FOR SOLVING THE ABOVE PROBLEM

In order to achieve the abovementioned object, in a first aspect of the present invention, an optical image measurement device comprises: a broadband light source that outputs a low coherent light (L₀), an interference-light generator configured to split a light from the broadband light source into a signal light and a reference light, and superimpose the signal light propagated through a measured object and the reference light propagated through a reference object to generate an interference light; a scanner configured to scan a position to radiate the signal light on the measured object; a detector configured to receive the interference light to convert into electric charges, and accumulate the electric charges to generate a detection signal; an image forming part configured to form an image of the measured object based on the detection signal; a determining part configured to determine whether an intensity of the detection signal is equal to or more than a predetermined threshold; and a controller configured to control at least one of a scan speed and scan position of the signal light by the scanner, and a time for accumulation of electric charges by the detector so as to increase the intensity of the detection signal of the interference light when it is determined that the intensity is less than the predetermined threshold, and the device is characterized in that the image forming part is configured to form an image of the measured object based on the detection signal with the increased intensity.

Further, in a second aspect of the present description, the optical image measurement device according to the first aspect is characterized in that: the detector is configured to receive the interference light to convert into electric charges, and accumulate the electric charges to generate a detection signal; and the controller is configured to control the detector so as to increase the time for accumulating the electric charges and an amount of the electric charges accumulated by the detector, thereby increasing the intensity of the detection signal of the interference light.

Further, in a third aspect of the present invention, the optical image measurement device according to the third aspect is characterized in that: the controller is configured to control the scanner to scan with the signal light at a scan speed corresponding to the increased accumulation time.

Further, in a fourth aspect of the present invention, the optical image measurement device according to the third aspect is characterized in that: the controller is configured to control the scanner to sequentially radiate the signal light to such a number of scan points that corresponds to the increased accumulation time.

Further, in a fifth aspect of the present invention, the optical image measurement device according to the second aspect is characterized in that the controller is configured to control the broadband light source to increase a time of output of the light from the broadband light source, thereby increasing the accumulation amount.

Further, in a sixth aspect of the present invention, the optical image measurement device the fifth aspect is characterized in that: the controller is configured to obtain a scan speed corresponding to the increased accumulation time, and to control the scanner to scan the signal light with the obtained scan speed.

Further, in an seventh aspect of the present invention, the optical image measurement device according to the fifth aspect is characterized in that: the controller is configured to obtain such a number of scan points that corresponds to the increased output time, and to control the scanner to sequentially radiate the signal light to the obtained number of scan points.

Further, in a eighth aspect of the present invention, the optical image measurement device according to the second aspect is characterized in that the controller is configured to control the broadband light source to increase an intensity of output of the light, from the broadband light source thereby increasing the accumulation amount.

Further, in a ninth aspect of the present invention, the optical image measurement device according to the eighth aspect is characterized in that: the controller is configured to control the scanner to scan with the signal light at a scan speed corresponding to the increased output intensity.

Further, in an tenth aspect of the present invention, the optical image measurement device according to the eighth aspect is characterized in that: the interference-light generator is provided with a scanner configured to scan a position to radiate the signal light on the measured object; and the controller is configured to control the scanner to sequentially radiate the signal light to such a number of scan points that corresponds to the increased output intensity.

Further, in a eleventh aspect of the present invention, the optical image measurement device according to the first aspect is characterized in that: the image forming part is provided with a calculator configured to obtain intensities of a plurality of frequency components of the detection signal; the determining part is configured to specify a minimum value of the intensities of the plurality of frequency components and determine whether the minimum value is equal to or more than a predetermined value, thereby determining the intensity of the detection signal; and the controller is configured to, when it is determined that the minimum value is less than the predetermined value, increase the intensity of the detection signal of the interference light so that the intensity of a frequency component corresponding to the minimum value becomes equal to or more than the predetermined value.

Further, in a twelfth aspect of the present invention, the optical image measurement device according to the first aspect is characterized in that: the determining part is configured to specify a maximum value of pixel values of a plurality of pixels of the image and determine whether the maximum value is equal to or more than a predetermined value, thereby determining the intensity of the detection signal; and the controller is configured to, when it is determined that the maximum value is less than the predetermined value, increase the intensity of the detection signal of the interference light so that the pixel value of a pixel corresponding to the maximum value becomes equal to or more than the predetermined value.

Further, in a thirteenth aspect of the present invention, the optical image measurement device according to the first aspect is characterized in that: the determining part is configured to specify pixels having pixel values equal to or more than a predetermined value from among a plurality of pixels of the image, and determine whether a number of the specified pixels is equal to or more than a predetermined number, thereby determining the intensity of the detection signal; and the controller is configured to, when it is determined that the number is less than the predetermined value, increase the intensity of the detection signal of the interference light so that the number of pixels having pixel values equal to or more than the predetermined value becomes equal to or more than the predetermined number.

Further, in a fourteenth aspect of the present invention, the optical image measurement device according to the first aspect is characterized in that: the determining part is configured to determine whether a new intensity of the detection signal of the interference light after the control by the controller is equal to or more than the predetermined threshold; and the controller is configured to, when it is determined that the new intensity is less than the predetermined threshold, control the broadband light source, the detector and the image forming part to form two or more images and superimpose the two or more images, thereby forming a new image.

Further, in a fifteenth aspect of the present invention, the optical image measurement device according to the first aspect is characterized in that: the determining part is configured to determine whether a new intensity of the detection signal after the control by the controller is equal to or more than the predetermined threshold; and the controller is configured to, when it is determined that the new intensity is less than the predetermined threshold, control the image forming part to execute predetermined image processing for increasing an image quality on the image.

Further, in a sixteenth aspect of the present invention, the optical image measurement device according to the first aspect is characterized in that: the determining part is configured to determine whether a new intensity of the detection signal of the interference light after the control by the controller is equal to or more than the predetermined threshold; and the controller is configured to, when it is determined that the new intensity is less than the predetermined threshold, control the image forming part to increase an amplitude of the detection signal to generate a new detection signal and form an image based on the new detection signal.

### EFFECT OF THE INVENTION

According to the present invention, when the intensity of a detection signal of an interference light is less than a predetermined threshold, the device acts to increase the intensity of the detection signal and form an image. Therefore, it is possible to acquire a clear image even when the intensity of the interference light is low.

### BRIEF DESCRIPTION OF THE DRAWINGS

Fig. 1 is a schematic configuration diagram showing an example of the entire configuration of an embodiment of a fundus oculi observation device functioning as an optical image measurement device according to the present invention.
Fig. 2 is a schematic configuration diagram showing an example of the configuration of a scan unit installed in a retinal camera unit in the embodiment of the fundus oculi observation device functioning as the optical image measurement device according to the present invention.
Fig. 3 is a schematic configuration diagram showing an example of the configuration of an OCT unit in the embodiment of the fundus oculi observation device functioning as the optical image measurement device according to the present invention.
Fig. 4 is a schematic block diagram showing an example of the hardware configuration of an arithmetic and control unit in the embodiment of the fundus oculi observation device functioning as the optical image measurement device according to the present invention.
Fig. 5 is a schematic block diagram showing an example of the configuration of a control system in the embodiment of the fundus oculi observation device functioning as the optical image measurement device according to the present invention.
Fig. 6 is a schematic block diagram showing an example of the configuration of a control system in the embodiment of the fundus oculi observation device functioning as the optical image measurement device according to the present invention.
Fig. 7 is a timing chart showing an example of a timing of control by the embodiment of the fundus oculi observation device functioning as the optical image measurement device according to the present invention.
Fig. 8 is a timing chart showing an example of a timing of control in the embodiment of the fundus oculi observation device functioning as the optical image measurement device according to the present invention.
Figs. 9A and 9B are schematic views showing an example of a scan pattern of a signal light in the embodiment of the fundus oculi observation device functioning as the optical image measurement device according to the present invention. Fig. 9A shows an example of the scan pattern of the signal light when the fundus oculi is seen from the incident side of the signal light into an eye. Fig. 9B shows an example of an arrangement pattern of scan points on each scan line.
Fig. 10 is a schematic view showing an example of the scan pattern of the signal light and a pattern of a tomographic image formed along each scan line in the embodiment of the fundus oculi observation device functioning as the optical image measurement device according to the present invention.
Fig. 11 is a flow chart showing an example of a usage pattern in the embodiment of the fundus oculi observation device functioning as the optical image measurement device according to the present invention.
Fig. 12 is a flow chart showing an example of a usage pattern in the embodiment of the fundus oculi observation device functioning as the optical image measurement device according to the present invention.
Fig. 13 is a flow chart showing an example of a usage pattern in the embodiment of the fundus oculi observation device functioning as the optical image measurement device according to the present invention.

### DESCRIPTION OF REFERENCE NUMERALS AND SYMBOLS

1 fundus oculi observation device (optical image measurement device)
1A retinal camera unit
141 scan unit
150 OCT unit
160 low-coherence light source
174 reference mirror
180 spectrometer
184 CCD
200 arithmetic and control unit
210 controller
220 image forming part
221 frequency-component calculator
230 image processor
231 intensity determining part
232 control setting part
233 output-intensity setting part
234 emission-time setting part
235 scan-speed setting part
236 scan-position setting part
237 accumulation-time setting part
238 data processor
240 user interface

### BEST MODE FOR CARRYING OUT THE INVENTION

An example of an embodiment of an optical image measurement device according to the present invention will be described in detail with reference to the drawings.

The optical image measurement device according to the present invention is a device that forms a tomographic image and a three-dimensional image of a measured object by using the OCT technique. A measurement method to be applied may be any method such as the Fourier Domain type, the Swept Source type, and the full-field type. In the case of execution of control relating to a scan with a signal light, any measurement method of scanning with a signal light such as the Fourier Domain type and the Swept Source type is applied.

### [DEVICE CONFIGURATION]

In this embodiment, a fundus oculi observation device that acquires an OCT image (a tomographic image, a three-dimensional image and so on) of the fundus oculi will be described. A fundus oculi observation device 1 functions as a Fourier Domain type of optical image measurement device. In this embodiment, a measured object is the fundus oculi.

### [ENTIRE CONFIGURATION]

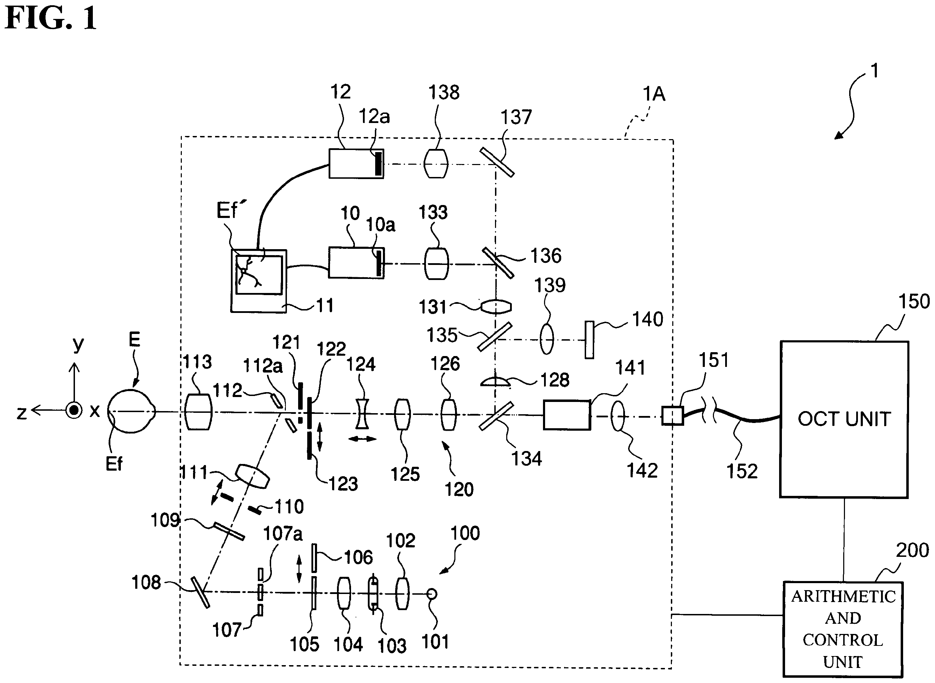

As shown in Fig. 1, the fundus oculi observation device 1 includes a retinal camera unit 1A, an OCT unit 150, and an arithmetic and control unit 200. The fundus camera unit 1A has almost the same optical system as a conventional retinal camera. A retinal camera is a device that captures a two-dimensional image of the fundus oculi surface. The OCT unit 150 houses an optical system for acquiring an OCT image. The arithmetic and control unit 200 is provided with a computer that executes various kinds of arithmetic processes, control processes, and so on.

To the OCT unit 150, one end of a connection line 152 is attached. A connector part 151 that connects the connection line 152 to the retinal camera unit 1A is attached to the other end of the connection line 152. An optical fiber runs through inside the connection line 152. Thus, the OCT unit 150 and the retinal camera unit 1A are optically connected via the connection line 152.

### [Configuration of Retinal Camera Unit]

The retinal camera unit 1A has an optical system for forming a two-dimensional image of the fundus oculi surface. A two-dimensional image of the fundus oculi surface represents, for example, a color image, a monochrome image and a fluorescent image (a fluorescein angiography image, an indocyanine green fluorescent image, and so on) obtained by imaging the fundus oculi surface. As well as a conventional retinal camera, the retinal camera unit 1A is provided with an illumination optical system 100 that illuminates a fundus oculi Ef, and an imaging optical system 120 that leads the fundus oculi reflected light of the illumination light to an imaging device 10.

The imaging device 10 of the imaging optical system 120 detects an illumination light having a wavelength of near-infrared region, the details of which will be described later. Moreover, the imaging optical system 120 is also provided with an imaging device 12 that detects an illumination light having a wavelength of visible region. Furthermore, the imaging optical system 120 acts to lead a signal light coming from the OCT unit 150 to the fundus oculi Ef and also lead the signal light propagated through the fundus oculi Ef to the OCT unit 150.

The illumination optical system 100 includes an observation light source 101, a condenser lens 102, an imaging light source 103, a condenser lens 104, exciter filters 105 and 106, a ring transparent plate 107, a mirror 108, an LCD (Liquid Crystal Display) 109, an illumination diaphragm 110, a relay lens 111, an aperture mirror 112, and an objective lens 113.

The observation light source 101 outputs an illumination light having a wavelength of visible region included in the range of about 400-700 nm, for example. On the other hand, the imaging light source 103 outputs an illumination light having a wavelength of near-infrared region included in the range of about 700-800 nm, for example. The near-infrared light outputted from the imaging light source 103 is set so as to have a shorter wavelength than a light used by the OCT unit 150 (described later).

Further, the imaging optical system 120 includes the objective lens 113, (an aperture 112a of) the aperture mirror 112, an imaging diaphragm 121, barrier filters 122 and 123, a magnifying lens 124, a relay lens 125, an imaging lens 126, a dichroic mirror 134, a field lens 128, a half mirror 135, a relay lens 131, a dichroic mirror 136, an imaging lens 133, the imaging device 10 (an image pick-up element 10a), a reflection mirror 137, an imaging lens 138, the imaging device 12 (an image pick-up element 12a), a lens 139, and an LCD 140.

Furthermore, the imaging optical system 120 is provided with the dichroic mirror 134, the half mirror 135, the dichroic mirror 136, the reflection mirror 137, the imaging lens 138, the lens 139, and the LCD 140.

The dichroic mirror 134 is configured to reflect the fundus oculi reflected light (having a wavelength included in the range of about 400-800 nm) of the illumination light coming from the illumination optical system 100, and to transmit a signal light LS (having a wavelength included in the range of about 800-900 nm, for example; described later) coming from the OCT unit 150.

Further, the dichroic mirror 136 is configured to transmit the illumination light having a wavelength of visible region coming from the illumination optical system 100 (a visible light having a wavelength of about 400-700 nm outputted from the observation light source 101), and to reflect the illumination light having a wavelength of near-infrared region (a near-infrared light having a wavelength of about 700-800 nm outputted from the imaging light source 103).

The LCD 140 displays a fixation target (an internal fixation target) for fixing an eye E. The light from the LCD 140 is focused by the lens 139, and thereafter, reflected by the half mirror 135 and propagated through the field lens 128 to be reflected by the dichroic mirror 136. Furthermore, this light is propagated through the imaging lens 126, the relay lens 125, the magnifying lens 124, the (aperture 112a of the) aperture mirror 112, the objective lens 113 and so on, and enters the eye E. Consequently, an internal fixation target is projected onto the fundus oculi Ef of the eye E.

The image pick-up element 10a is an image pick-up element such as a CCD (Charge Coupled Device) or a CMOS (Complementary Metal Oxide Semiconductor) installed in the imaging device 10 such as a TV camera, and specifically detects a light having a wavelength of near-infrared region. In other words, the imaging device 10 is an infrared TV camera that detects a near-infrared light. The imaging device 10 outputs a video signal as the result of detection of the near-infrared light.

A touch panel monitor 11 displays a two-dimensional image of the surface of the fundus oculi Ef (a fundus oculi image Ef') based on the video signal. Further, this video signal is transmitted to the arithmetic and control unit 200, and the fundus oculi image is displayed on a display (described later).

For imaging by the imaging device 10, for example, an illumination light having a wavelength of near-infrared region outputted from the imaging light source 103 is used.

On the other hand, the image pick-up element 12a is an image pick-up element such as a CCD or a CMOS installed in the imaging device 12 such as a TV camera, and specifically detects a light having a wavelength of visible region. In other words, the imaging device 12 is a TV camera that detects a visible light. The imaging device 12 outputs a video signal as the result of detection of the visible light.

The touch panel monitor 11 displays a two-dimensional image of the surface of the fundus oculi Ef (the fundus oculi image Ef') based on the video signal. Further, this video signal is transmitted to the arithmetic and control unit 200, and the fundus oculi image is displayed on the display (described later).

For imaging the fundus oculi by the imaging device 12, for example, an illumination light having a wavelength of visible region outputted from the observation light source 101 is used.

The retinal camera unit 1A is provided with a scan unit 141 and a lens 142. The scan unit 141 scans a position on the fundus oculi Ef radiated by a light outputted from the OCT unit 150 (the signal light LS; described later). The scan unit 141 is an example of the "scanner" of the present invention.

The lens 142 collimates the signal light LS led from the OCT unit 150 through the connection line 152, and makes the light enter the scan unit 141. Further, the lens 142 focuses the fundus oculi reflected light of the signal light LS propagated through the scan unit 141.

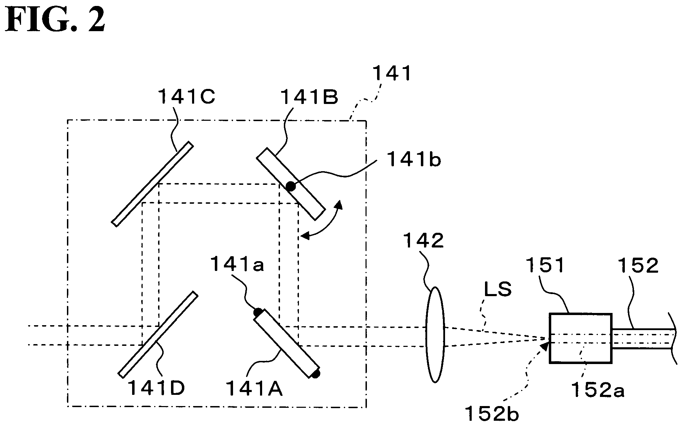

Fig. 2 shows an example of the configuration of the scan unit 141. The scan unit 141 includes Galvano mirrors 141A and 141B, and reflection mirrors 141C and 141D.

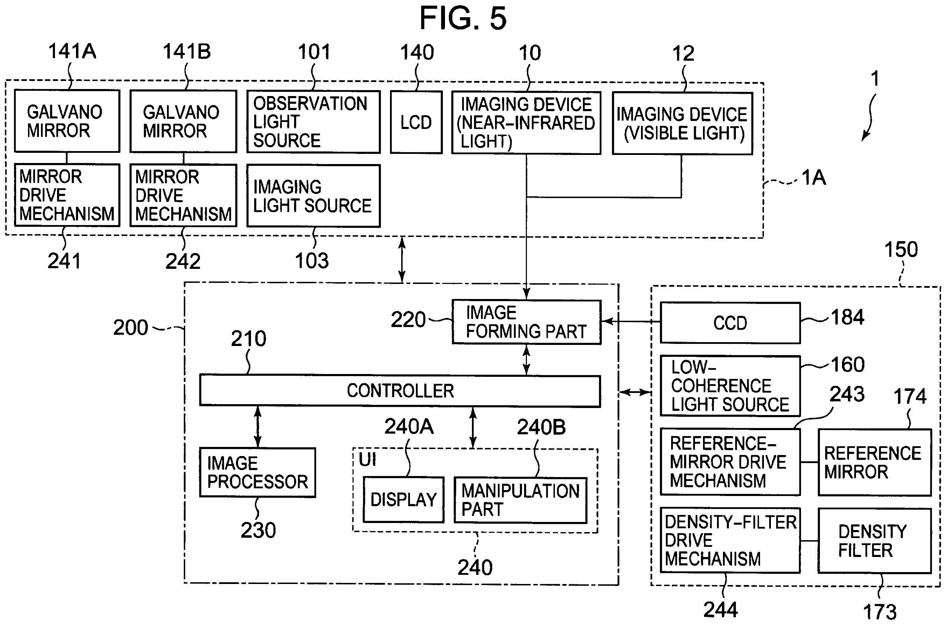

The Galvano mirrors 141A and 141B are reflection mirrors arranged so as to be rotatable about rotary shafts 141a and 141b, respectively. The respective Galvano mirrors 141A and 141B are rotated about the rotary shafts 141a and 141b by drive mechanisms described later (mirror drive mechanisms 241 and 242 shown in Fig. 5). Thus, the directions of the reflection surfaces (surfaces to reflect the signal light LS) of the respective Galvano mirrors 141A and 141B are changed.

The rotary shafts 141a and 141b are arranged orthogonally to each other. In Fig. 2, the rotary shaft 141a of the Galvano mirror 141A is arranged in the parallel direction to the paper surface. On the other hand, the rotary shaft 141b of the Galvano mirror 141B is arranged in the orthogonal direction to the paper surface.

That is to say, the Galvano mirror 141B is configured to be rotatable in the direction indicated by an arrow pointing to both directions in Fig. 2, whereas the Galvano mirror 141A is configured to be rotatable in the direction orthogonal to the arrow pointing to both the directions. Consequently, the Galvano mirrors 141A and 141B act to change the reflection directions of the signal light LS into directions orthogonal to each other, respectively. As apparent from Figs. 1 and 2, a scan with the signal light LS is performed in the x-direction when the Galvano mirror 141A is rotated, and a scan with the signal light LS is performed in the y-direction when the Galvano mirror 141B is rotated.

The signal light LS reflected by the Galvano mirrors 141A and 141B is reflected by the reflection mirrors 141C and 141D, and travels in the same direction as having entered the Galvano mirror 141A.

An end surface 152b of an optical fiber 152a inside the connection line 152 is arranged so as to face the lens 142. The signal light LS emitted from the end surface 152b travels while expanding the beam diameter thereof toward the lens 142, and is collimated by the lens 142. On the contrary, the signal light LS propagated through the fundus oculi Ef is focused to the end surface 152b by the lens 142, and enters the optical fiber 152a.

### [Configuration of Oct Unit]

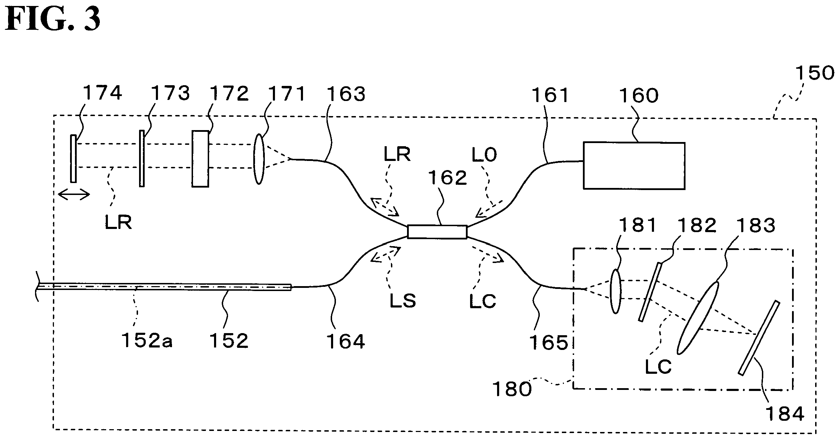

Next, the configuration of the OCT unit 150 will be described with reference to Fig. 3. The OCT unit 150 has an optical system for forming an OCT image of the fundus oculi.

The OCT unit 150 is provided with an optical system substantially the same as that of a conventional optical image measurement device. That is to say, the OCT unit 150 splits a low-coherence light into a reference light and a signal light, superimposes the signal light propagated through an eye and the reference light propagated through a reference object, generates an interference light, and detects this interference light. This detection result (a detection signal) is inputted into the arithmetic and control unit 200. The arithmetic and control unit 200 analyzes this detection signal and forms a tomographic image or a three-dimensional image of the fundus oculi.

A low-coherence light source 160 is composed of a broadband light source that outputs a low-coherence light L0. As the broadband light source, for example, a super luminescent diode (SLD), a light emitting diode (LED) or the like is used. The low-coherence light source 160 is an example of the "light source" of the present invention.

The low-coherence light L0 is, for example, a light that includes a light having a wavelength of near-infrared region and has a temporal coherence length of about several tens of micrometers. The low-coherence light L0 has a longer wavelength than the illumination light (having a wavelength of about 400-800 nm) of the retinal camera unit 1A, for example, a wavelength included in the range of about 800-900 nm.

The low-coherence light L0 outputted from the low-coherence light source 160 is led to an optical coupler 162 through an optical fiber 161. The optical fiber 161 is composed of, for example, a single mode fiber, a PM (polarization maintaining) fiber or the like. The optical coupler 162 splits the low-coherence light L0 into a reference light LR and the signal light LS.

The optical coupler 162 acts as both a part for splitting a light (a splitter) and a part for superposing lights (a coupler), but will be herein referred to as an "optical coupler" idiomatically.

The reference light LR generated by the optical coupler 162 is led by an optical fiber 163 composed of a single mode fiber or the like, and is emitted from the end surface of the fiber. Further, the reference light LR is collimated by a collimator lens 171, and thereafter, propagated through a glass block 172 and a density filter 173 and reflected by a reference mirror 174. The reference mirror 174 is an example of the "reference object" of the present invention.

The reference light LR reflected by the reference mirror 174 is again propagated through the density filter 173 and the glass block 172, focused to the fiber end surface of the optical fiber 163 by the collimator lens 171, and guided to the optical coupler 162 through the optical fiber 163.

Here, the glass block 172 and the density filter 173 act as a delaying part for matching the optical path lengths (the optical distances) of the reference light LR and the signal light LS, and also as a dispersion compensating part for matching the dispersion properties of the reference light LR and the signal light LS.

Further, the density filter 173 also acts as a neutral density filter that reduces the light amount of the reference light LR. The density filter 173 is composed of, for example, a rotary-type ND (Neutral Density) filter. The density filter 173 is driven to rotate by a drive mechanism (a density-filter drive mechanism 244 described later; refer to Fig. 5) including a driver such as a motor. Consequently, the light amount of the reference light LR contributing to generation of the interference light LD is changed.

Further, the reference mirror 174 is configured to be movable in the traveling direction (the direction of an arrow pointing to both sides shown in Fig. 3) of the reference light LR. Thus, it is possible to ensure the optical path length of the reference light LR according to the axial length of the eye E, a working distance (a distance between the objective lens 113 and the eye E) and so on. Moreover, by moving the reference mirror 174, it is possible to acquire an image at any depth position of the fundus oculi Ef. The reference mirror 174 is moved by a drive mechanism (a reference-mirror drive mechanism 243 described later; refer to Fig. 5) including a driver such as a motor.

On the other hand, the signal light LS generated by the optical coupler 162 is guided to the end of the connection line 152 through an optical fiber 164 composed of a single mode fiber or the like. The optical fiber 152a runs through inside the connection line 152. Here, the optical fiber 164 and the optical fiber 152a may be composed of a single optical fiber, or may be integrally formed by joining the end surfaces of the respective fibers, for example. Anyway, it is sufficient as far as the optical fibers 164 and 152a are configured to be capable of transmitting the signal light LS between the retinal camera unit 1A and the OCT unit 150.

The signal light LS is led through the inside of the connection line 152 and guided to the retinal camera unit 1A. Furthermore, the signal light LS is propagated through the lens 142, the scan unit 141, the dichroic mirror 134, the imaging lens 126, the relay lens 125, the magnifying lens 124, the imaging diaphragm 121, the aperture 112a of the aperture mirror 112 and the objective lens 113, and radiated to the eye E. For radiating the signal light LS to the eye E, the barrier filters 122 and 123 are previously retracted from the optical path, respectively.

The signal light LS having entered the eye E is formed into an image on the fundus oculi Ef and then reflected. At this moment, the signal light LS not only is reflected by the surface of the fundus oculi Ef but also reaches a deep region of the fundus oculi Ef to be scattered at the refractive index boundary. Therefore, the signal light LS propagated through the fundus oculi Ef contains information reflecting the surface morphology of the fundus oculi Ef and information reflecting the state of backscatter at the refractive index boundary of deep layer tissues of the fundus oculi Ef. This light may be simply referred to as the "fundus oculi reflected light of the signal light LS."

The fundus oculi reflected light of the signal light LS travels reversely on the abovementioned path in the retinal camera unit 1A to be focused to the end surface 152b of the optical fiber 152a, enters the OCT unit 150 through the optical fiber 152a, and returns to the optical coupler 162 through the optical fiber 164.

The optical coupler 162 superimposes the signal light LS having returned through the eye E and the reference light LR reflected by the reference mirror 174 to generate an interference light LC. This interference light LC is led to a spectrometer 180 through an optical fiber 165 composed of a single mode fiber or the like.

Although a Michelson-type interferometer is employed in this embodiment, it is possible to properly employ any type of interferometer such as the Mach-Zehnder-type.

The "interference-light generator" of the present invention includes, for example, the optical coupler 162, an optical member on the optical path of the signal light LS (namely, an optical member placed between the optical coupler 162 and the eye E), and an optical member on the optical path of the reference light LR (namely, an optical member placed between the optical coupler 162 and the reference mirror 174). To be specific, the interference-light generator includes an interferometer provided with the optical coupler 162, the optical fibers 163 and 164 and the reference mirror 174.

The spectrometer 180 includes a collimator lens 181, a diffraction grating 182, an image forming lens 183, and a CCD 184. The diffraction grating 182 may be a transmission-type diffraction grating that transmits light, or may be a reflection-type diffraction grating that reflects light. Moreover, it is also possible to use another photodetecting device such as a CMOS device, instead of the CCD 184.

The interference light LC having entered the spectrometer 180 is collimated by the collimator lens 181, and divided into spectra by the diffraction grating 182 (spectral resolution). The divided interference light LC are formed into an image on the image pick-up surface of the CCD 184 by the image forming lens 183. The CCD 184 detects the respective spectral components of the divided interference light LC and converts into electric charges. The CCD 184 accumulates the electric charges and generates a detection signal. Furthermore, the CCD 184 transmits the detection signal to the arithmetic and control unit 200. The time and timing of accumulation of the electric charges, and also the timing of transmission of the detection signal are controlled by the arithmetic and control unit 200, for example. The CCD 184 is an example of the "detector" of the present invention.

### [Configuration of Arithmetic and Control Unit]

Next, the configuration of the arithmetic and control unit 200 will be described. The arithmetic and control unit 200 analyzes the detection signal inputted from the CCD 184 of the OCT unit 150, and forms an OCT image of the fundus oculi Ef. An analysis method here is the same as in a conventional technique of the Fourier domain OCT.

Further, the arithmetic and control unit 200 forms a two-dimensional image showing the morphology of the surface of the fundus oculi Ef based on the video signals outputted from the imaging devices 10 and 12 of the retinal camera unit 1A.

Furthermore, the arithmetic and control unit 200 controls each part of the retinal camera unit 1A and the OCT unit 150.

The arithmetic and control unit 200 executes control of the retinal camera unit 1A such as: control of output of the illumination lights by the observation light source 101 and the imaging light source 103; control of insertion/retraction of the exciter filters 105, 106 and the barrier filters 122, 123 to/from the optical path; control of operation of a display device such as the LCD 140; control of movement of the illumination diaphragm 110 (control of the diaphragm value); control of the diaphragm value of the imaging diaphragm 121; and control of movement of the magnifying lens 124 (control of the magnification). Furthermore, the arithmetic and control unit 200 executes control of operation of the Galvano mirrors 141A and 141B.

On the other hand, the arithmetic and control unit 200 executes control of the OCT unit 150 such as: control of output of the low-coherence light L0 by the low-coherence light source 160; control of movement of the reference mirror 174; control of rotation operation of the density filter 173 (operation of changing the reduction amount of the light amount of the reference light LR); and control of the timing of accumulation and the timing of output of signals by the CCD 184.

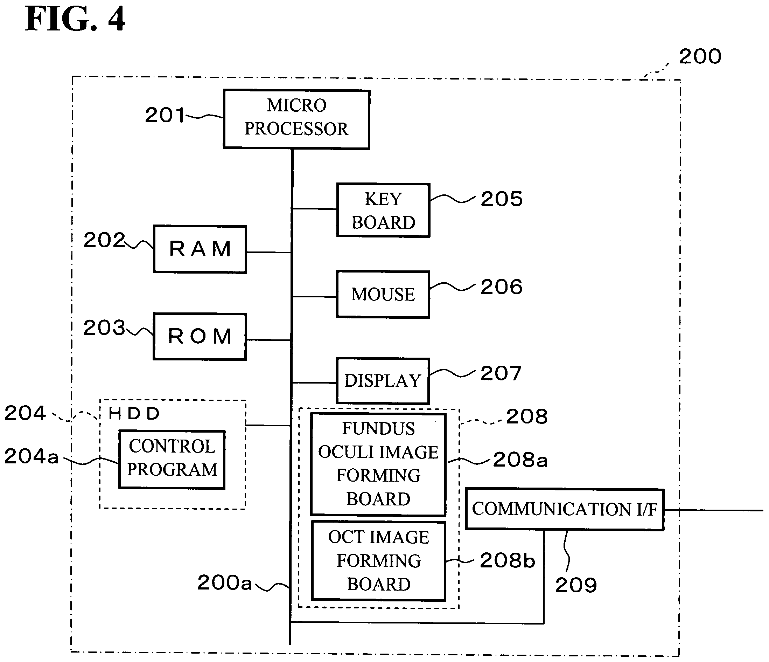

The hardware configuration of this arithmetic and control unit 200 will be described with reference to Fig. 4.

The arithmetic and control unit 200 is provided with the same hardware configuration as a conventional computer. To be specific, the arithmetic and control unit 200 includes a microprocessor 201, a RAM 202, a ROM 203, a hard disk drive (HDD) 204, a keyboard 205, a mouse 206, a display 207, an image forming board 208, and a communication interface (I/F) 209. The respective parts are connected by a bus 200a.

The microprocessor 201 includes a CPU (Central Processing Unit), an MPU (Micro Processing unit) or the like. The microprocessor 201 reads out a control program 204a from the hard disk drive 204 and loads onto the RAM 202, thereby causing the fundus oculi observation device 1 to execute an operation characteristic to the present embodiment.

Further, the microprocessor 201 executes control of each of the aforementioned parts of the device, various kinds of arithmetic processes, and so on. Moreover, the microprocessor 201 receives an operation signal from the keyboard 205 or the mouse 206 and, in accordance with the content of the operation, controls each of the parts of the device. Furthermore, the microprocessor 201 executes control of a display process by the display 207, control of a process of transmission/reception of data and signals by the communication interface 209, and so on.

The keyboard 205, the mouse 206, and the display 207 are used as user interfaces of the fundus oculi observation device 1. For example, the keyboard 205 is used as a device for typing letters, figures or the like. The mouse 206 is used as a device for performing various kinds of input operations to the display screen of the display 207.

Further, the display 207 is a display device such as an LCD or a CRT (Cathode Ray Tube) display, and displays various kinds of images such as an image of the fundus oculi Ef formed by the fundus oculi observation device 1, and also displays various kinds of screens such as an operation screen and a set-up screen.

The user interface of the fundus oculi observation device 1 is not limited to such a configuration, and may include, for example, a trackball, a joystick, a touch-panel LCD, and a control panel for ophthalmic examination. As the user interface, it is possible to employ any configuration provided with a function of displaying/outputting information and a function of inputting information and manipulating the device.

The image forming board 208 is a dedicated electronic circuit that executes a process of forming (image data of) an image of the fundus oculi Ef. The image forming board 208 is provided with a fundus oculi image forming board 208a and an OCT image forming board 208b.

The fundus oculi image forming board 208a is a dedicated electronic circuit that forms image data of a fundus oculi image based on video signals from the imaging device 10 and the imaging device 12.

On the other hand, the OCT image forming board 208b is a dedicated electronic circuit that forms image data of a tomographic image of the fundus oculi Ef based on a detection signal coming from the CCD 184 of the OCT unit 150.

By installing this image forming board 208, it is possible to increase the processing speed for the process of forming a fundus oculi image and a tomographic image.

The communication interface 209 transmits control signals from the microprocessor 201, to the retinal camera unit 1A or the OCT unit 150. Moreover, the communication interface 209 receives video signals from the imaging devices 10 and 12 and a detection signal from the CCD 184 of the OCT unit 150, and inputs them into the image forming board 208. At this moment, the communication interface 209 inputs the video signals from the imaging devices 10 and 12, into the fundus oculi image forming board 208a, and inputs the detection signal from the CCD 184, into the OCT image forming board 208b.

Further, in a case that the arithmetic and control unit 200 is connected to a communication line such as a LAN (Local Area Network) or the Internet, it is possible to provide the communication interface 209 with a network adapter such as a LAN card or communication equipment such as a modem, thereby configuring to be capable of data communication via this communication network. In this case, it is possible to install a server that stores the control program 204a to the communication network and configure the arithmetic and control unit 200 as a client terminal of the server, thereby causing the fundus oculi observation device 1 to operate.

### [Configuration of Control System]

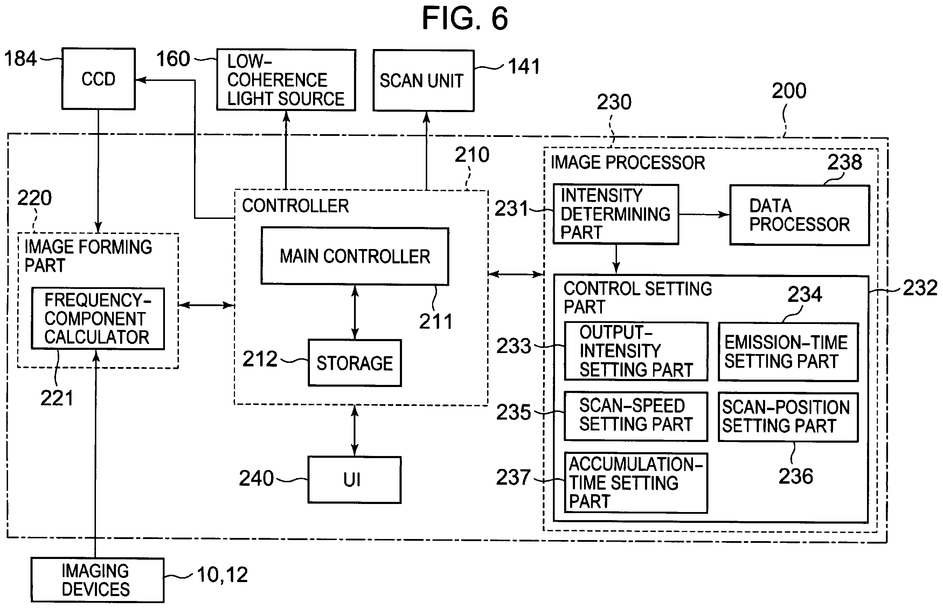

Next, the configuration of a control system of the fundus oculi observation device 1 will be described with reference to Figs. 5 and 6.

### (Controller)

The control system of the fundus oculi observation device 1 is configured mainly by a controller 210 of the arithmetic and control unit 200. The controller 210 includes the microprocessor 201, the RAM 202, the ROM 203, the hard disk drive 204 (the control program 204a), the communication interface 209, and so on.

The controller 210 is provided with a main controller 211 and a storage 212. The main controller 211 executes the aforementioned various kinds of controls.

The storage 212 stores various kinds of data. The data stored in the storage 212 is, for example, the image data of an OCT image, the intensity of a detection signal (the intensity of each frequency component), subject information (information on a subject such as the patient ID and name), and so on. The main controller 211 executes a process of writing the data into the storage 212, and a process of reading out the data from the storage 212.

### (Image Forming Part)

An image forming part 220 forms the image data of the fundus oculi image Ef' based on the video signals from the imaging devices 10 and 12.

Further, the image forming part 220 forms the image data of a tomographic image of the fundus oculi Ef based on the detection signal from the CCD 184. This process includes, for example, noise elimination (noise reduction), filtering, FFT (Fast Fourier Transform), and so on. To be specific, a frequency-component calculator 221 executes FFT, thereby obtaining the intensities of frequency components of a detection signal. The frequency components of the detection signal correspond to the spectral components of the interference light LC. The frequency-component calculator 221 is an example of the "calculator" of the present invention.

For example, the image forming part 220 determines the pixel value (the luminance value) based on the intensity of a detection signal, more specifically, the intensities of frequency components, thereby forming the image data of an OCT image. Thus, the detection signal (the intensity) and the image data (the pixel value) have a correspondence relation with each other. In this specification, the intensity of the detection signal and the pixel value of the image data may be identified with each other.

The image forming part 220 includes the image forming board 208, the communication interface 209, and so on. In this specification, "image data" and an "image" displayed based thereon may be identified with each other.

### (Image Processor)

An image processor 230 executes various kinds of image processing and analysis processes on the image data of an image formed by the image forming part 220. For example, the image processor 230 executes various kinds of correction processes such as luminance correction and dispersion correction of an image. Moreover, the image processor 230 also executes a process relating to a detection signal (frequency components) as described later.

Further, the image processor 230 executes an interpolation process of interpolating pixels between tomographic images formed by the image forming part 220, thereby forming the image data of a three-dimensional image of the fundus oculi Ef.

The image data of a three-dimensional image means such image data that the positions of the pixels are defined by the three-dimensional coordinate system. An example of the image data of a three-dimensional image is image data composed of three-dimensionally arranged voxels. This image data is referred to as volume data, voxel data, or the like. For displaying an image based on volume data, the image processor 230 executes a rendering process (such as volume rendering and MIP (Maximum Intensity Projection)) on this volume data, and forms the image data of a pseudo three-dimensional image seen from a specific view direction. On a display device such as the display 207, this pseudo three-dimensional image based on the image data is displayed.

Further, it is also possible to form stack data of a plurality of tomographic images as the image data of a three-dimensional image. Stack data is image data obtained by three-dimensionally arranging a plurality of tomographic images obtained along a plurality of scan lines based on the positional relation of the scan lines.

An intensity determining part 231 of the image processor 230 determines whether the intensity of a detection signal is equal to or more than a predetermined threshold. In other words, the intensity determining part 231 determines whether the pixel values of pixels of the image data of an OCT image are equal to or more than the predetermined threshold. Here, "being equal to or more than the predetermined threshold" means that being equal to the predetermined threshold or more than the predetermined threshold. However, in the former, it is not necessary to be strictly equal to the predetermined threshold, and a difference shall be allowed to the extent that the actions and effects of this embodiment are achieved. This is the same for the term "less than." The intensity determining part 231 is an example of the "determining part" of the present invention. Below, examples of processes executed by the intensity determining part 231 will be described.

A first process example is based on the frequency components of a detection signal. A detection signal includes a plurality of frequency components. The intensity of each of the frequency components is acquired by the frequency-component calculator 221. The intensity determining part 231 compares the intensities of the plurality of frequency components and specifies the minimum value.

Next, the intensity determining part 231 determines whether this minimum value is equal to or more than a predetermined value. This predetermined value is previously set. The intensity determining part 231 determines that the intensity of the detection signal is equal to or more than the predetermined threshold when the minimum value is equal to or more than the predetermined value. On the contrary, when the minimum value is less than the predetermined value, it is determined that the intensity of the detection signal is less than the predetermined threshold.

A second process example is based on the pixel values (the luminance values) of pixels of an OCT image (a tomographic image). The intensity determining part 231 compares the pixel values of a plurality of pixels of an OCT image and specifies the maximum value.

Next, the intensity determining part 231 determines whether this maximum value is equal to or more than a predetermined value. This predetermined value is previously set. The intensity determining part 231 determines that the intensity of the detection signal is equal to or more than the predetermined threshold when the maximum value is equal to or more than the predetermined value. On the contrary, when the maximum value is less than the predetermined value, it is determined that the intensity of the detection signal is less than the predetermined threshold.

A third process example is also based on the pixel values (the luminance values) of pixels of an OCT image (a tomographic image). The intensity determining part 231 specifies a pixel whose pixel value is equal to or more than a predetermined value from among a plurality of pixels of the OCT image. This process can be executed by comparing the pixel value of each of the pixels with the predetermined value and determining whether the pixel value is equal to or more than the predetermined value. This predetermined value is previously set.

Next, the intensity determining part 231 determines whether the number of the pixels having pixel values equal to or more than the predetermined value is equal to or more than a predetermined number. This process can be executed by counting the number of the pixels that have pixel values equal to or more than the predetermined value and comparing the number with the predetermined number. Moreover, similar determination may be executed by generating a histogram of pixel values in an OCT image, specifying the number of pixels having pixel values equal to or more than the predetermined value, and comparing the number with the predetermined number. This predetermined number is previously set. The intensity determining part 231 determines that the intensity of the detection signal is equal to or more than the predetermined threshold when the number of the pixels having pixel values equal to or more than the predetermined value is equal to or more than the predetermined number. On the contrary, when this number is less than the predetermined number, it is determined that the intensity of the detection signal is less than the predetermined threshold.

A control setting part 232 operates when the intensity determining part 231 determines that the intensity of the detection signal is less than the predetermined threshold. The control setting part 232 sets various kinds of control contents for increasing the intensity of the detection signal. The control contents include, for example, the output intensity and emission time of the low-coherence light L0, the scan speed and scan position of the signal light LS, and the time of accumulation of electric charges by the CCD 184.

An output-intensity setting part 233 sets the intensity (the brightness) of the low-coherence light L0 outputted by the low-coherence light source 160. In a case that the optical image measurement device is applied in the medical field such as the ophthalmic field, the maximum value of the intensity of the low-coherence light is preset, and it is prohibited to set the intensity more than this maximum value. This is for avoiding a situation that the low-coherence light L0 (the signal light LS) damages a patient and for performing a measurement with safety. Below, examples of a process of setting the output intensity of the low-coherence light will be described.

In a first process example, the output-intensity setting part 233 sets a new output intensity by increasing the output intensity of the low-coherence light L0 that is the base of the detection signal determined by the intensity determining part 231 (the original output intensity), by a predetermined amount. The increase amount at this moment is preset.

In a second process example, the output-intensity setting part 233 sets a new output intensity of the low-coherence light L0 based on the difference between the intensity of the detection signal and the predetermined threshold.

For example, in a case that the first process example of the intensity determining part 231 is applied, the output-intensity setting part 233, when the minimum value of the frequency components of the detection signal is less than the predetermined value, subtracts the minimum value from the predetermined value, and sets a new output intensity based on the value of this difference. This process can be executed by, for example, previously generating and storing information that relates the values of the differences and the increase amounts, and determining the increase amount based on this information. Alternatively, the increase amount may be calculated based on the relation between the minimum value and the value of the difference.

In a case that the second process example of the intensity determining part 231 is applied, the output-intensity setting part 233, when the maximum value of the pixel values is less than the predetermined value, subtracts the maximum value from the predetermined value and sets a new output intensity based on the value of this difference. This process can be executed by, for example, previously generating and storing information that relates the values of the differences and the increase amounts, and determining the increase amount based on this information. Alternatively, the increase amount may be calculated based on the relation between the maximum value and the value of the difference.

In a case that the third process example of the intensity determining part 231 is applied, the output-intensity setting part 233, when the number of the pixels having pixel values equal to or more than the predetermined value, subtracts the number from the predetermined number and sets a new output intensity based on the value of this difference. This process can be executed by, for example, previously generating and storing information that relates the values of the differences and the increase amounts, and determining the increase amount based on this information. Alternatively, the increase amount may be calculated based on the relation between the number and the value of the difference.

An emission-time setting part 234 sets the output time (the emission time) of the low-coherence light L0 by the low-coherence light source 160. The emission-time setting part 234 sets the emission time as in the aforementioned setting of the output intensity, for example. As in the case of the output intensity, it is prohibited to set such an emission time that exceeds the maximum amount of light that can be radiated to a human body. This maximum light amount is set in accordance with, for example, the output intensity.

A scan-speed setting part 235 sets the scan speed of the signal light LS. Here, it is possible to define the scan speed as the speed (the dimension is distance/time) of scanning the signal light LS to the fundus oculi Ef, and it is also possible to define the scan speed as the time interval (namely, the measurement time interval) for moving the signal light LS to the adjacent scan point (described later).

The scan-speed setting part 235 can set the scan speed with reference to other setting contents. For example, the scan-speed setting part 235 sets the scan speed with reference to the output intensity set by the output-intensity setting part 233. This process is executed based on, for example, information that relates the output intensity and the scan speed. The scan speed is set so that, for example, the accumulation amount of light radiated to the eye E remains within a safe range.

Further, the scan-speed setting part 235 sets the scan speed with reference to the emission time set by the emission-time setting part 234. At this moment, the scan speed (the measurement time interval) is set so as to synchronize with the emission time.

A scan-position setting part 236 sets the scan position of the signal light LS. The scan position is set as the position of a scan point as described later (a scan line is also determined based on the arrangement of the scan points).

The scan-position setting part 236 can set the scan position with reference to other setting contents. For example, the scan-position setting part 236 sets the scan position with reference to the output intensity set by the output-intensity setting part 233. In this process, for example, by determining the number of the scan points so that the accumulation amount of light radiated to the eye E remains within a safe range, and arranging these scan points at equal intervals, the scan positions are set.

Further, the scan-position setting part 236 sets the scan positions with reference to the emission time set by the emission-time setting part 234. In this process, for example, by determining the number of the scan points based on the scan time (pre)determined in consideration of the influence of displacement due to eye movement and based on the emission time, and arranging these scan points at equal intervals, the scan positions are set.

An accumulation-time setting part 237 sets the time of accumulation of electric charges by the CCD 184. Below, an example of this process will be described. As in the aforementioned process of setting the output intensity, the accumulation-time setting part 237 can increase the accumulation time by a preset amount, or can calculate the increase amount of the accumulation time based on the difference between the intensity of a detection signal and the predetermined threshold.

The scan-speed setting part 235 can set the scan speed of the signal light LS with reference to the accumulation time set by the accumulation-time setting part 237. Moreover, the scan-position setting part 236 can set the scan position of the signal light LS based on the set accumulation time. The scan speed and the scan position are set so as to synchronize with the accumulation time.

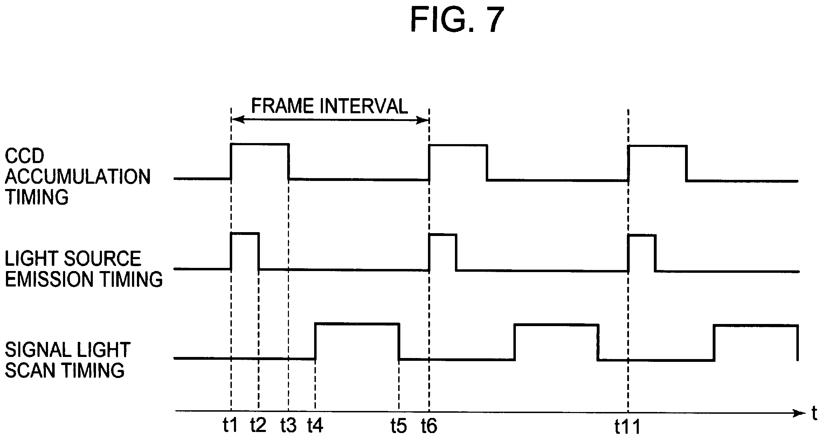

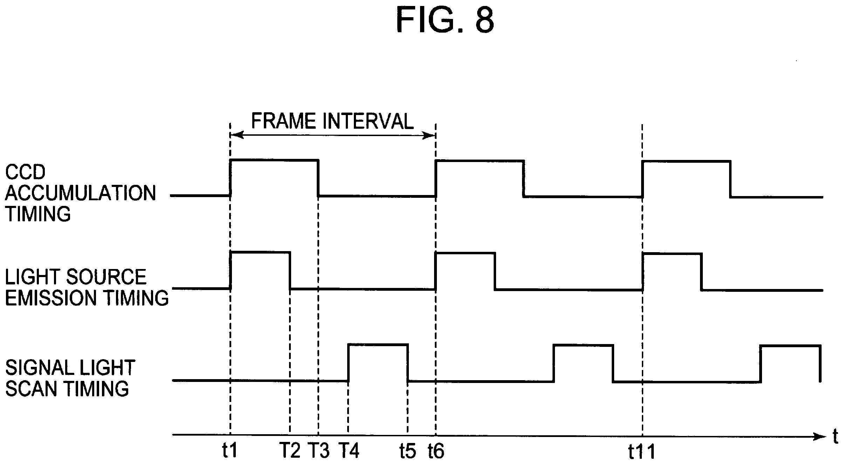

Here, the control timings by the scan unit 141, the low-coherence light source 160 and the CCD 184 will be described with reference to Figs. 7 and 8. Here, a timing chart shown in Fig. 7 represents the control timing before the control content is changed. A timing chart shown in Fig. 8 represents the control content having been changed. In this example, frame intervals shall be identical to each other before and after the change.

In the control timings shown in Fig. 7, firstly, a measurement of a first scan point is started at time t1. That is to say, at time t1, the low-coherence light source 160 is controlled to start output of the low-coherence light L0, and simultaneously, the CCD 184 is controlled to start accumulation of electric charges. A slight gap may exist between these control timings. For example, it is possible to control the CCD 184 slightly before controlling the low-coherence light source 160, thereby allowing leeway so as not to fail to detect the low-coherence light L0 when starting the output.

Next, at time t2, the low-coherence light source 160 is controlled to stop the output of the low-coherence light L0.

Subsequently, at time t3, the CCD 184 is controlled to stop the accumulation of electric charges. The accumulation of electric charges by the CCD 184 has finished at time t2 in fact, but in these control timings, the accumulation of electric charges is stopped at time t3 after time t2 to allow leeway so as not to fail to detect the low-coherence light L0 right before stopping the output. This is the end of the measurement of the first scan point.

Next, between time t4 and time t5, the scan unit 141 is controlled to move the radiation position of the signal light LS. Consequently, the radiation position of the signal light LS is moved to a second scan point.

Then, at time t6, the output of the low-coherence light L0 is started, and the accumulation of electric charges is also started, whereby a measurement of a second scan point is started. Here, by executing the controls at the same timings as described above, the measurement of the second scan point is executed. Likewise, at time t11, a measurement of a third scan point is started. The frame interval of these control timings is t(k+5)-tk(k=1,2...).

Assuming the intensity of the detection signal obtained by executing the measurements at such control timings is equal to or less than the predetermined threshold, the control setting part 232 sets new control timings in accordance therewith.

Assuming the control setting part 232 has set the emission time of the low-coherence light L0 and furthermore has set the scan speed of the signal light LS in accordance with this new emission time, the control timings shown in Fig. 8 have been set in accordance therewith.

In the control timings shown in Fig. 8, at time t1 identical to that before the change, a measurement of the first scan point is started at first. That is to say, at time t1, the output of the low-coherence light L0 is started, and the accumulation of electric charges is also started. The gap between the control timings is also identical to that before the change.

Next, at time T2 different from the time before the change, the low-coherence light 160 is controlled to stop the output of the low-coherence light L0. Here, T2 is larger than t2. That is to say, since the emission-time setting part 234 sets the emission time of the low-coherence light L0 longer than that before the change, T2-t1 is larger than t2-t1, and accordingly T2 is larger than t2.

Subsequently, at time T3 different from the time before the change, the CCD 184 is controlled to stop the accumulation of electric charges. Although the accumulation of electric charges has been finished at time T2 in fact, the accumulation of electric charges is stopped at time T3 after time T2 so that leeway is allowed. An accumulation time T3-t1 after the change is set by the accumulation-time setting part 237 so as to be longer than an accumulation time t3-t1 before the change (that is, T3>t3). Here, in a case that the accumulation time t3 before the change is set after time T2, it is not necessary to newly set the accumulation time. This is the end of the measurement of the first scan point.

Next, at time T4 different from the time before the change, the scan unit 141 is controlled to start the movement of the radiation position of the signal light LS. Time T4 is set after time T2, and moreover, after time T3.

Subsequently, at time t5 identical to that before the change, the scan unit 141 is controlled to stop the movement of the radiation position of the signal light LS. Consequently, the radiation position of the signal light LS is moved to the second scan point.

Here, assuming the distance between the first scan point and the second scan point are identical between before and after the change, in consideration that a movement time after the change t5-T4 is shorter than a movement time before the change t5-t4 (t5-T4 < t5-t4), the scan speed after the change needs to be faster than that before the change. In order that this condition is satisfied, the scan-speed setting part 235 sets a new scan speed based on an emission time after the change (and an accumulation time after the change).

Next, at time t6 identical to that before the change, a measurement of the second scan point is started. Here, the controls are executed at the same timings as described before, whereby the measurement of the second scan point is executed. Likewise, at time t11, a measurement of the third scan point is started.

Although the case of newly setting the scan speed has been described in the above example, the same is applied in the case of newly setting the scan positions. In the case of not changing the scan speed, it is possible to, in accordance with extension of the emission time and so on, set some of the scan points before the change (for example, every other point) as new scan points, or set some of the scan lines before the change (for example, every other line) as new scan lines. Alternatively, both the scan speed and the scan positions may be changed.

Further, also in a case that the output intensity of the low-coherence light L0 is increased, it is possible to newly set the scan speed, the scan positions and the accumulation time in accordance with the increased amount as in the above example. Furthermore, it is also possible to change the emission time in accordance with the increased amount of the output intensity.

A data processor 238 processes image data and a detection signal. Examples of processes executed by the data processor 238 will be described below.

In a first process example, the data processor 238 generates an image (a superimposed image) by superimposing two or more images obtained by measuring (substantially) the same position on the fundus oculi Ef. This superimposed image is generated by superimposing two or more images along the same scan line, for example.

The image superimposing process is executed in the following manner, for example. Firstly, position matching of images is executed as needed. This process is executed by, for example, searching a characteristic region such as a vascular cross section or a morphologically characteristic site (the macula or the like) from each of the images and matching the position of this characteristic region. Moreover, in a case that each of the images is part of a three-dimensional image, it is possible to execute position matching by using an accumulated image obtained by accumulating the three-dimensional images along the depth direction of the fundus oculi Ef (for example, refer to Japanese Unexamined Patent Application Publication No. 2007-130403). Through this position matching, two or more images are associated with each other by pixel unit.

When the position matching of the images is finished, the pixel values (the luminance values or the like) of two or more associated pixels are summed. At this moment, the value of the sum of the pixel values is divided by the number of the associated pixels (namely, the number of the superimposed images) as needed.

New image data is generated from pixels having the pixel value thus obtained. This new image data is image data of the superimposed image described above. Consequently, a superimposed image with sharpness and high luminance is obtained.

In a second process example, the data processor 238 executes predetermined image processing on an OCT image. This image processing is, for example, averaging, filtering, and so on. Averaging is a process of averaging the distribution of the pixel values in an image. For example, when averaging is executed on a luminance image, the distribution of brightness in the image is uniformized. Further, filtering is a process of correcting the pixel value of a certain pixel based on the pixel values of the surrounding pixels. By executing such image processing, it is possible to increase the quality of an image.

In a third process example, the data processor 238 executes signal processing for increasing the amplitude of a detection signal. This signal processing is, for example, a process of eliminating noise included in a detection signal. This also includes a process of eliminating part of the noise included in the detection signal, namely, a noise reduction process. A new detection signal obtained by eliminating the noise is sent to the image forming part 220. The image forming part 220 forms an OCT image (a tomographic image) based on this new detection signal.

The image processor 230 described above includes the microprocessor 201, the RAM 202, the ROM 203, the hard disk drive 204 (the control program 204a) and so on.

The image forming part 220 and the image processor 230 (specifically, the data processor 238) are included in the "image forming part" of the present invention. Moreover, the controller 210 and the control setting part 232 are included in the "controller" of the present invention.

### (User Interface)

The user interface (UI) 240 is provided with a display 240A and a manipulation part 240B. The display 240A is composed of a display device such as the display 207. Moreover, the manipulation part 240B is composed of an input device and a manipulation device such as the keyboard 205 and the mouse 206.

### (Scan with Signal Light and Image Processing)

An example of the scan pattern of the signal light LS and the image processing pattern will be described. A scan with the signal light LS is executed by the scan unit 141. To be specific, the scan with the signal light LS is executed by control of the mirror drive mechanisms 241 and 242 by the controller 210 to change the directions of the reflecting surfaces of the Galvano mirrors 141A and 141B.

The Galvano mirror 141A scans with the signal light LS in the horizontal direction (the x-direction in Fig. 1). The Galvano mirror 141B scans with the signal light LS in the vertical direction (the y-direction in Fig. 1). Further, by operating both the Galvano mirrors 141A and 141B simultaneously, it is possible to scan with the signal light LS in any direction on the xy-plane.

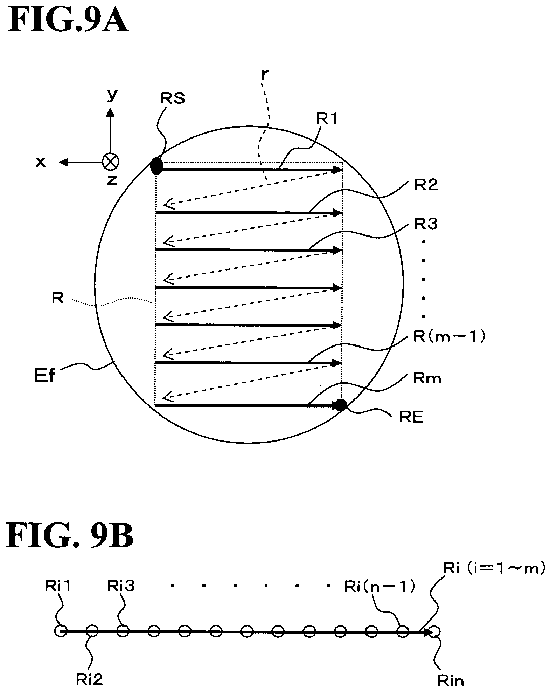

Figs. 9A and 9B show an example of the scan pattern of the signal light LS for forming an image of the fundus oculi Ef. Fig. 9A shows an example of the scan pattern of the signal light LS, when the fundus oculi Ef is seen from a direction that the signal light LS enters the eye E (that is, seen from -z side toward +z side in Fig. 1). Further, Fig. 9B shows an example of an arrangement pattern of the scan points (measurement positions) on each scan line on the fundus oculi Ef.

As shown in Fig. 9A, a scan with the signal light LS is executed within a rectangular scan region R. Within this scan region R, a plurality of (m lines of) scan lines R1-Rm along the x-direction are set. Scan lines Ri (i=1-m) are arranged in the y-direction. A direction of each of the scan lines Ri (the x-direction) will be referred to as the "main scan direction" and a direction orthogonal thereto (the y-direction) will be referred to as the "sub-scan direction."

On each of the scan lines Ri, as shown in Fig. 9B, a plurality of (n pieces of) scan points Ri1-Rin are set. The positions of the scan region R, scan line Ri and scan point Rij are properly set before execution of a measurement.

In order to execute the scan shown in Figs. 9A and 9B, the controller 210 firstly controls the Galvano mirrors 141A and 141B to set the incident target of the signal light LS into the fundus oculi Ef to a scan start position RS (a scan point R11) on the first scan line R1. Subsequently, the controller 210 controls the low-coherence light source 160 to flash the low-coherence light L0, thereby making the signal light LS enter the scan start position RS. The CCD 184 receives the interference light LC based on the reflected light of this signal light LS at the scan start position RS, accumulates electric charges, and generates a detection signal.

Next, the controller 210 controls the Galvano mirror 141A to scan with the signal light LS in the main scan direction to set the incident target to a scan point R12, and flashes the low-coherence light L0 to make the signal light LS enter the scan point R12. The CCD 184 receives the interference light LC based on the reflected light of this signal light LS at the scan point R12, accumulates electric charges, and generates a detection signal.

Likewise, the controller 210 controls to generate a detection signal corresponding to each of the scan points, by flashing the low-coherence light L0 at each of the scan points while sequentially moving the incident target of the signal light LS from a scan point R13 to R14, ----, R1(n-1), and R1n.

When the measurement at a last scan point R1n on the first scan line R1 is finished, the controller 210 simultaneously controls the Galvano mirrors 141A and 141B to move the incident target of the signal light LS to a first scan point R21 on a second scan line R2 following a line switching scan r. Then, the controller 210 controls to execute the same measurement on each of scan points R2j (j=1-n) on this second scan line R2 and to generate a detection signal corresponding to each of the scan points R2j.

Likewise, the controller 210 controls to execute a measurement on each of a third scan line R3, ----, an m-1th scan line R(m-1), an mth scan line Rm and to generate a detection signal corresponding to each scan point. Symbol RE on the scan line Rm is a scan end position corresponding to a scan point Rmn.

Thus, the controller 210 controls to generate m×n pieces of detection signals corresponding to m×n pieces of scan points Rij (i=1-m, j=1-n) within the scan region R. A detection signal corresponding to each of the scan points Rij may be denoted by Dij.

In the above control, when the Galvano mirrors 141A and 141 B are operated, the controller 210 acquires position information of each of the scan points Rij (coordinates in the xy coordinate system). This position information (scan position information) is referred to when an OCT image is formed, for example.

Next, an example of image processing when the scan shown in Fig. 9 is executed will be described.

The image forming part 220 forms tomographic images of the fundus oculi Ef along the respective lines Ri (the main scan direction). Moreover, the image processor 230 forms a three-dimensional image of the fundus oculi Ef based on the tomographic images formed by the image forming part 220.

The tomographic image forming process includes a two-step arithmetic process as conventional. In the first step, based on each detection signal Dij, an image in the depth direction (the z-direction in Fig. 1) of the fundus oculi Ef at the scan point Rij is formed.

In the second step, the depthwise images at the scan points Ri1-Rin are arranged based on the scan position information, and a tomographic image Gi along the scan line Ri is formed. Through the above process, m pieces of tomographic images G1-Gm are obtained.

The image processor 230 arranges the tomographic images G1-Gm based on the scan position information and executes an interpolating process of interpolating an image between the adjacent tomographic images Gi and G(i+1), thereby generating a three-dimensional image of the fundus oculi Ef. This three-dimensional image is defined by the three-dimensional coordinate system (x,y,z) based on the scan position information, for example.

Further, the image processor 230 can form a tomographic image at any cross-section, based on this three-dimensional image. When the cross-section is designated, the image processor 230 specifies the position of each scan point (and/or an interpolated depthwise image) on the designated cross-section, extracts a depthwise image (and/or an interpolated depthwise image) at each specified position from the three-dimensional image, and arranges a plurality of extracted depthwise images based on the scan position information and so on, thereby forming a tomographic image at the designated cross-section.

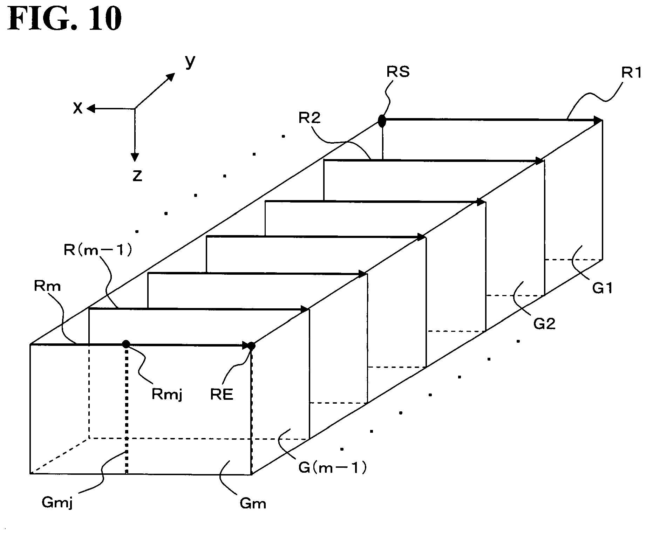

An image Gmj shown in Fig. 10 represents a depthwise image at the scan point Rmj on the scan line Rm. Likewise, a depthwise image at the scan point Rij formed in the aforementioned first-step is represented as an "image Gij."

The scan pattern of the signal light LS by the fundus oculi observation device 1 is not limited to the abovementioned one. For example, it is possible to scan with the signal light LS only in the horizontal direction (the x-direction), only in the perpendicular direction (the y-direction), in the longitudinal and lateral directions like a cruciform, radially, circularly, concentrically, or helically. That is to say, as mentioned before, the scan unit 141 is configured to be capable of independently scanning with the signal light LS in the x-direction and the y-direction, so that it is possible to scan with the signal light LS along any trajectory on the xy-plane.

### [USAGE PATTERN]

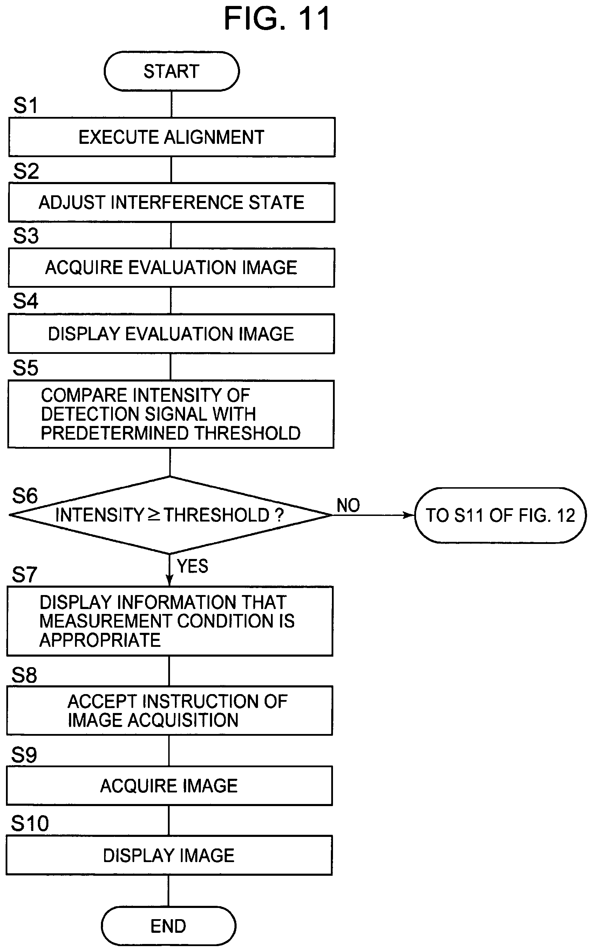

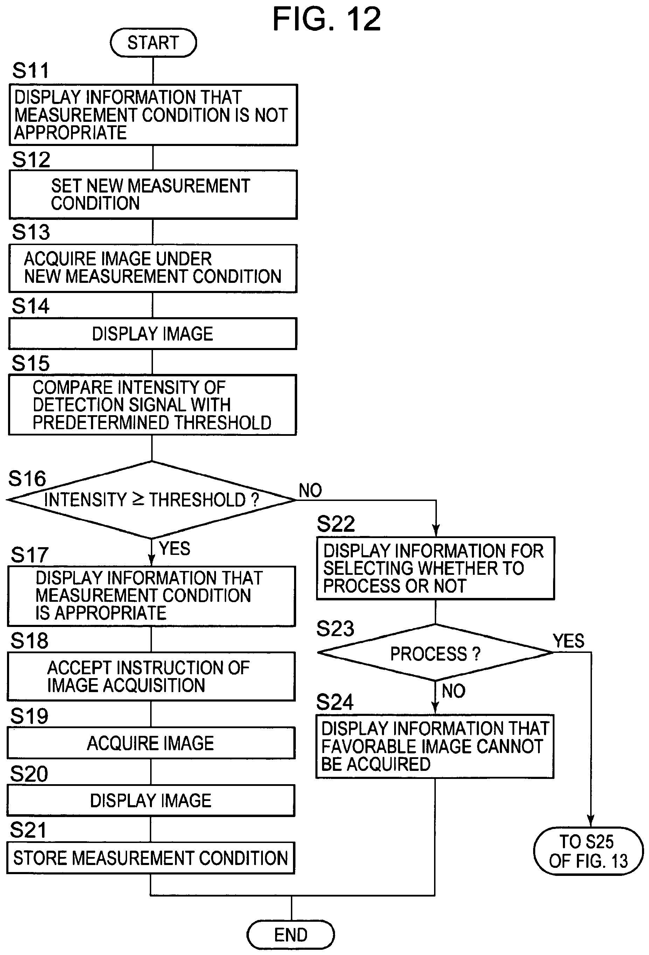

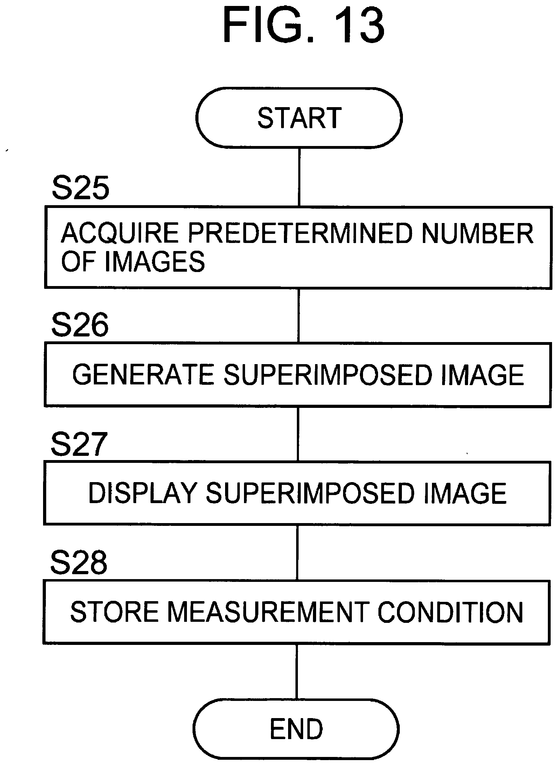

A usage pattern of the fundus oculi observation device 1 will be described. Flow charts shown in Figs.11-13 are examples of the usage pattern of the fundus oculi observation device 1.

Firstly, alignment of an optical system with the eye E is executed (S1). The alignment is executed as in a conventional retinal camera. For example, by adjusting the position of the retinal camera unit 1A while projecting an alignment bright point (not shown) to the eye E to observe the state thereof, the alignment is executed.

Next, the position of the reference mirror 174 is adjusted, and the interference state of the signal light and the reference light is adjusted (S2). At this moment, the adjustment is executed so that an image at a desired depth position of the fundus oculi Ef becomes clear. The position adjustment of the reference mirror 174 may be manually performed by using the manipulation part 240B, or may be automatically performed.

Next, an image of the fundus oculi Ef is acquired (S3). At this moment, a measurement is executed by applying the control pattern shown in the timing chart of Fig. 7 and the scan pattern shown in Fig. 9. This image is for evaluating whether a favorable image is obtained under the applied measurement condition (referred to as an evaluation image). The main controller 211 controls the display 240A to display this evaluation image (S4).

Based on the detection signal (the frequency components) of the interference light LC or the evaluation image, the intensity determining part 231 compares the intensity of the detection signal with a predetermined threshold and determines the size relationship thereof (S5).

### [When Intensity of Detection Signal is Equal to or More Than Predetermined Threshold]

When it is determined that the intensity of the detection signal is equal to or more than the predetermined threshold (S6; Y), the main controller 211 controls the display 240A to display information that the measurement condition is appropriate (S7). This displayed information is, for example, an OK mark, a predetermined message, or the like. Moreover, the evaluation image or the like may be displayed in a predetermined color (for example, blue or green). Moreover, it is possible to output audio information.

The operator instructs to acquire an image by using the manipulation part 240B (S8). This instruction is performed by pressing down an imaging button as in a conventional retinal camera, for example. In response to this instruction, the main controller 211 controls to execute a measurement under the same measurement condition as for the evaluation image and to acquire an image (S9). Then, the main controller 211 controls the display 240A to display this image (S10). This is the end of the process in this case. This image is used for observation by a doctor or the like.

### [When Intensity of Detection Signal is Less Than Predetermined Threshold]

On the other hand, when it is determined that the intensity of the detection signal is less than the predetermined threshold (S6; N), the main controller 211 controls the display 240A to display information that the measurement condition is not appropriate (S11). This displayed information is, for example, a message such as "signal is weak," a numerical value representing the signal intensity, and so on. Moreover, the evaluation image or the like may be displayed in a predetermined color (for example, red). Moreover, it is possible to output audio information.

Further, the control setting part 232 sets a new measurement condition (S12). At this moment, the control pattern shown in the timing chart of Fig. 8 shall be set. In a case that the scan positions are newly set, the scan pattern shown in Fig. 9 is also changed. However, the arrangement pattern of the scan lines is not changed in general (the number of the scan lines may be changed).

The main controller 211 controls to execute a measurement under this new measurement condition and to acquire an image (S13). Furthermore, the main controller 211 controls the display 240A to display this image (S14).

Based on the detection signal (the frequency components) or image obtained under the new measurement condition, the intensity determining part 231 compares the intensity of the detection signal with the predetermined threshold and determines the level relation thereof (S15).

When it is determined that the intensity of the detection signal is equal to or more than the predetermined threshold (S16; Y), the main controller 211 controls the display 240A to display information that the measurement condition is appropriate (S17).

The operator instructs acquisition of an image by using the manipulation part 240B (S18). In response to this instruction, the main controller 211 controls to execute a measurement under the measurement condition and to acquire an image (S19). Then, the main controller 211 controls the display 240A to display this image (S20). This image is used for observation by a doctor or the like.

The main controller 211 controls the storage 212 to store the measurement condition (and the scan pattern) together with patient information of the subject (S21). The destination to store the information may be another storage device. Thus, by storing the measurement condition and so on, it is possible to automatically reproduce the measurement condition and so on in a future examination. This is the end of the process in this case.

On the other hand, when it is determined that the intensity of the detection signal is less than the predetermined threshold (S16; N), the main controller 211 controls the display 240A to display information for selecting whether to process the image or not (S22).

The operator manipulates the manipulation part 240B to select whether to process or not (S23). When it is determined not to process (S23; N), the main controller 211 controls the display 240A to display information that a favorable image cannot be acquired (S24). This is the end of the process in this case.

A case of generating a superimposed image as data processing (S23) will be described. In this case, at step 22, it is possible to set the number of images to be superimposed. Further, the control setting part 232 may newly set a measurement condition as needed.

The main controller 211 controls the low-coherence light source 160, the scan unit 141, the CCD 184 and so on to acquire a predetermined number of images (S25). The data processor 238 superimposes these images to generate a superimposed image (S26).

The main controller 211 controls the display 240A to display this superimposed image (S27). Moreover, the main controller 211 controls the storage 212 to store the measurement condition (and the scan pattern) together with patient information of the subject (S28). This is the end of the process in this case.

In the case of executing image processing as data processing, the main controller 211 controls to acquire a new image, and the data processor 238 executes image processing on this new image.

Further, in the case of increasing the amplitude of the detection signal as data processing, the main controller 211 controls to execute a new measurement, the data processor 238 increases the amplitude of the detection signal having been thereby obtained to generate a new detection signal, and the image forming part 220 forms an image based on this new detection signal.

### [ACTIONS AND EFFECTS]

The actions and effects of the fundus oculi observation device 1 as described above will be described.

The fundus oculi observation device 1 functions as an optical image measurement device configured to split the low-coherence light L0 into the signal light LS and the reference light LR, superimpose the signal light LS propagated through the fundus oculi Ef and the reference light LR propagated through the reference mirror 174 to generate the interference light LC, detect the interference light LC to generate a detection signal, and form an image of the fundus oculi Ef based on this detection signal. Furthermore, the fundus oculi observation device 1 determines whether the intensity of the detection signal of the interference light LC is equal to or more than a predetermined threshold and, when it is determined that the intensity is less than the predetermined threshold, executes control to increase the intensity of the detection signal of the interference light LC.

The interference light LC is detected by the CCD 184. The CCD 184 receives the interference light LC to convert into electric charges, and accumulates the electric charges to generate a detection signal. The controller 210 executes control to increase the amount of the electric charges accumulated by the CCD 184, thereby increasing the intensity of the detection signal.

To be specific, the fundus oculi observation device 1 executes controls to increase the accumulation amount of the electric charges, such as: (1) increase the accumulation time of electric charges by the CCD 184; (2) increase the output time of the low-coherence light L0; and (3) increase the output intensity of the low-coherence light. The contents of control may be set individually, or may be set in conjunction with each other.

Further, there are processes of determining the intensity of the detection signal as described below. As a first determining process, there is a method of determining the intensity of the detection signal by determining whether the minimum value of the intensities of the frequency signals of the detection signal is equal to or more than a predetermined value. In this case, in a case that it is determined that the minimum value is less than the predetermined value, it is desirable to increase the intensity of the detection signal of the interference light LC so that the frequency component corresponding to the minimum value becomes equal to or more than the predetermined value. In a case that it is impossible to increase the intensity in the above manner, it is possible to execute the aforementioned data processing.

As a second determining process, there is a method of determining the intensity of the detection signal by determining whether the maximum value of the pixel values of pixels composing the image is equal to or more than a predetermined value. In this case, when it is determined that the maximum value is less than the predetermined value, it is desirable to increase the intensity of the detection signal of the interference light LC so that the pixel value of the pixel corresponding to the maximum value becomes equal to or more than the predetermined value. In a case that it is impossible to increase the intensity in the above manner, it is possible to execute the aforementioned data processing.

As a third determining process, there is a method of determining the intensity of the detection signal by specifying pixels having pixel values equal to or more than a predetermined value from among the pixels composing the image, and determining whether the number of these pixels is equal to or more than a predetermined number. In this case, when it is determined that this number is less than the predetermined number, it is desirable to increase the intensity of the detection signal of the interference light LC so that the number of these pixels becomes equal to or more than the predetermined number. In a case that it is impossible to increase the intensity in the above manner, it is possible to execute the aforementioned data processing.

Further, the fundus oculi observation device 1 determines whether the intensity of a detection signal obtained after the new control content is set is equal to or more than a predetermined threshold. When it is determined that this new intensity is less than the predetermined threshold, the fundus oculi observation device 1 superimposes two or more images to form a superimposed image. The superimposed image is a clearer image than each of the original images. Moreover, by executing predetermined image processing on an image or executing signal processing for increasing the amplitude of the detection signal, it is possible to make the image clearer.

According to this fundus oculi observation device 1, even when the intensity of the interference light LC is low, it is possible to increase the intensity of the detection signal of the interference light LC, and therefore, it is possible to acquire a clear image.

In a case that it is impossible to sufficiently increase the intensity of the detection signal, it is possible to, for example, form a superimposed image, execute image processing, or increase the amplitude of the detection signal, thereby making the image clearer.

### [MODIFICATION]

The configuration described above is merely an example for favorably implementing the optical image measurement device relating to the present invention.

In the above embodiment, the output intensity and emission time of the low-coherence light L0, the scanning speed and scanning position of the signal light LS, the accumulation time by the CCD 184, and so on are automatically set. However, it is possible to configure to allow manual setting of the above. In this case, a screen for setting the above is displayed on the display 240A. The operator controls the manipulation part 240B to manually set a desired control content. It is also possible to configure to, after the setting contents are automatically set as in the above embodiment, display the setting contents so that it is possible to manually change.

In the above embodiment, a superimposed image is formed when it is impossible to make the intensity of the detection signal of the interference light sufficient even when the intensity is increased. However, it is also possible to configure to form a superimposed image in other situations.

For example, when it is determined that the intensity of the detection signal corresponding to an evaluation image is less than a predetermined threshold, it is possible to form two or more images and superimposing these images to form a superimposed image. With such a configuration, even when the intensity of the detection signal is not sufficient, it is possible to make the image clearer.

Further, in the above embodiment, predetermined image processing (averaging or filtering or the like) is executed when it is impossible to make the intensity of the detection signal of the interference light sufficient even when the intensity is increased. However, it is also possible to execute the image processing in other situations.

For example, when it is determined that the intensity of the detection signal corresponding to the evaluation image is less than the predetermined threshold, it is possible to execute the image processing on the image (the evaluation image or a newly acquired image). With such a configuration, it is possible to make the image clearer even when the intensity of the detection signal is not sufficient.

Further, in the above embodiment, signal processing for increasing the amplitude of the detection signal is executed when it is impossible to make the intensity of the detection signal of the interference light sufficient even when the intensity is increased. However, it is also possible to configure to execute the signal processing in other situations.

For example, when it is determined that the intensity of the detection signal corresponding to the evaluation image is less than the predetermined threshold, it is possible to execute the signal processing on the detection signal (the detection signal corresponding to the evaluation image or a newly acquired detection signal). With such a configuration, it is possible to make the image clearer even when the intensity of the detection signal is not sufficient.

In the case of acquiring an image of an eye, it may be impossible to increase the light amount of the signal light LS. For example, in various types of ophthalmic devices, the upper limit value of the light amount of light radiated to an eye is preset. This is for preventing damage to the retina and excessive glare.

In such a case, it is possible to increase the intensity of the detection signal of the interference light LC by increasing the light amount of the reference light LR. In this case, the light amount of the signal light LS is set constant (the light amount may be increased within a range not exceeding the upper limit value). The light amount of the reference light LR can be changed by controlling the density filter (ND filter) 173. This control process is executed by the main controller 211, for example.

When the light amount of the reference light LR is increased, the amplitude of the interference light LC is increased, and the intensity of the detection signal generated by the CCD 184 is thereby increased. At this moment, the intensities of background light components (direct current components) of the interference light LC are also increased. Therefore, there is a need to increase the light amount of the interference light LC so that the maximum light amount of the interference light LC does not exceed the dynamic range of the CCD 184.

Thus, by increasing the light amount of the reference light LR to increase the intensity of the detection signal of the interference light LC, even when the intensity of the interference light is low, it is possible to acquire a clear image while preventing an adverse effect on a subject. This modification can also be applied to a measured object other than an eye.

In the above embodiment, the position of the reference mirror 174 is changed to change the difference in optical path length between the optical path of the signal light LS and the optical path of the reference light LR. However, the method for changing the difference in optical path length is not limited thereto. For example, by integrally moving the retinal camera unit 1A and the OCT unit 150 with respect to the eye E to change the optical path length of the signal light LS, it is possible to change the difference in optical path length. To be specific, in a case that a measured object is not a living body, it is also possible to change the difference in optical path length by moving the measured object in the depth direction (the z-direction).

Further, in the above embodiment, a device that acquires an OCT image of the fundus oculi has been described. However, it is possible to apply the configuration of the above embodiment to a device capable of acquiring an OCT image of another site of an eye, such as the cornea. Moreover, the present invention can also be applied to an optical image measurement device that measures OCT images of various kinds of measured objects other than an eye. For example, the optical image measurement device according to the present invention can be applied to any field such as the engineering field and the biological field.

The control program 204a in the above embodiment can be stored into any storage medium that can be read by the driver of a computer. As this storage medium, for example, it is possible to use an optical disk, an magneto optical disk (CD-ROM, DVD-RAM, DVD-ROM, MO or the like), a magnetic storage medium (hard disk, floppy™ disk, ZIP or the like). Moreover, it is also possible to store into a storage device such as a hard disk drive or a memory. Furthermore, it is also possible to transmit this program through a network such as the Internet or LAN. The present invention is set out in the claims that follow. The embodiments, aspects or examples according to the present description that do not fall within the scope of said claims are provided for illustrative purposes only and do not form part of the present invention.

## Claims

1. An optical image measurement device, comprising:
a broadband light source (160) that outputs a low-coherence light;
an interference-light generator configured to split the light from the broadband light source into a signal light and a reference light, and superimpose the signal light propagated through a measured object and the reference light propagated through a reference object to generate an interference light;
a scanner (141) configured to scan a position to radiate the signal light on the measured object;
a detector (184) configured to receive the interference light to convert into electric charges, and accumulate the electric charges to generate a detection signal;
an image forming part (220) configured to form an image of the measured object based on the detection signal;
a determining part (231) configured to determine whether an intensity of the detection signal is equal to or more than a predetermined threshold;
**characterized in that**
the device further comprises:
a controller (210) configured to control at least one of a scan speed and scan position of the signal light by the scanner, and a time for accumulation of electric charges by the detector so as to increase the intensity of the detection signal of the interference light when it is determined that the intensity is less than the predetermined threshold,
wherein the image forming part (220) is configured to form an image of the measured object based on the detection signal with the increased intensity.

2. The optical image measurement device according to Claim 1, wherein:
the controller (210) is configured to control the scanner (141) to scan with the signal light at a scan speed corresponding to the increased accumulation time.

3. The optical image measurement device according to Claim 1, wherein:
the controller (210) is configured to control the scanner (141) to sequentially radiate the signal light to such a number of scan points that corresponds to the increased accumulation time.

4. The optical image measurement device according to Claim 3,
wherein the controller (210) is configured to control the broadband light source (160) to increase a time of output of the light from the broadband light source (160), thereby increasing the accumulation amount.

5. The optical image measurement device according to Claim 4, wherein:
the controller (210) is configured to obtain a scan speed corresponding to the increased accumulation time, and to control the scanner (141) to scan the signal light with the obtained scan speed.

6. The optical image measurement device according to Claim 4, wherein:
the controller (210) is configured to obtain such a number of scan points that corresponds to the increased output time, and to control the scanner (141) to sequentially radiate the signal light to the obtained number of scan points.

7. The optical image measurement device according to Claim 1,
wherein the controller (210) is configured to control the broadband light source (160) to increase an intensity of output of the light from the broadband light source (160), thereby increasing the accumulation amount.

8. The optical image measurement device according to Claim 7, wherein:
the controller (210) is configured to control the scanner (141) to scan with the signal light at a scan speed corresponding to the increased output intensity.

9. The optical image measurement device according to Claim 7, wherein:
the interference-light generator is provided with a scanner (141) configured to scan a position to radiate the signal light on the measured object; and
the controller (210) is configured to control the scanner (141) to sequentially radiate the signal light to such a number of scan points that corresponds to the increased output intensity.

10. The optical image measurement device according to Claim 1, wherein:
the image forming part (220) is provided with a calculator (221) configured to obtain intensities of a plurality of frequency components of the detection signal;
the determining part (231) is configured to specify a minimum value of the intensities of the plurality of frequency components and determine whether the minimum value is equal to or more than a predetermined value, thereby determining the intensity of the detection signal; and
the controller (210) is configured to, when it is determined that the minimum value is less than the predetermined value, increase the intensity of the detection signal of the interference light so that the intensity of a frequency component corresponding to the minimum value becomes equal to or more than the predetermined value.

11. The optical image measurement device according to Claim 1, wherein:
the determining part (231) is configured to specify a maximum value of pixel values of a plurality of pixels of the image and determine whether the maximum value is equal to or more than a predetermined value, thereby determining the intensity of the detection signal; and
the controller (210) is configured to, when it is determined that the maximum value is less than the predetermined value, increase the intensity of the detection signal of the interference light so that the pixel value of a pixel corresponding to the maximum value becomes equal to or more than the predetermined value.

12. The optical image measurement device according to Claim 1, wherein:
the determining part (231) is configured to specify pixels having pixel values equal to or more than a predetermined value from among a plurality of pixels of the image, and determine whether a number of the specified pixels is equal to or more than a predetermined number, thereby determining the intensity of the detection signal; and
the controller (210) is configured to, when it is determined that the number is less than the predetermined value, increase the intensity of the detection signal of the interference light so that the number of pixels having pixel values equal to or more than the predetermined value becomes equal to or more than the predetermined number.

13. The optical image measurement device according to Claim 1, wherein:
the determining part (231) is configured to determine whether a new intensity of the detection signal of the interference light after the control by the controller (210) is equal to or more than the predetermined threshold; and
the controller (210) is configured to, when it is determined that the new intensity is less than the predetermined threshold, control the broadband light source (160), the detector (184) and the image forming part (220) to form two or more images and superimpose the two or more images, thereby forming a new image.

14. The optical image measurement device according to Claim 1, wherein:
the determining part (231) is configured to determine whether a new intensity of the detection signal after the control by the controller (210) is equal to or more than the predetermined threshold; and
the controller (210) is configured to, when it is determined that the new intensity is less than the predetermined threshold, control the image forming part (220) to execute predetermined image processing for increasing an image quality on the image.

15. The optical image measurement device according to Claim 1, wherein:
the determining part (231) is configured to determine whether a new intensity of the detection signal of the interference light after the control by the controller (210) is equal to or more than the predetermined threshold; and
the controller (210) is configured to, when it is determined that the new intensity is less than the predetermined threshold, control the image forming part (220) to increase an amplitude of the detection signal to generate a new detection signal and form an image based on the new detection signal.

## Patentansprüche

1. Optische Bildmessvorrichtung, umfassend:
eine Breitbandlichtquelle (160), die ein Licht mit niedriger Kohärenz ausgibt;
einen Interferenzlichterzeuger, der konfiguriert ist, das Licht von der Breitbandlichtquelle in ein Signallicht und ein Bezugslicht zu teilen und das Signallicht, das durch ein Messobjekt propagiert wird, und das Bezugslicht, das durch ein Bezugsobjekt propagiert wird, zu überlagern, um ein Interferenzlicht zu erzeugen;
einen Scanner (141), der konfiguriert ist, eine Position zu scannen, um das Signallicht auf das Messobjekt auszustrahlen;
einen Detektor (184), der konfiguriert ist, das Interferenzlicht zu empfangen, um es in elektrische Ladungen umzuwandeln und die elektrischen Ladungen anzusammeln, um ein Detektionssignal zu erzeugen;
einen Bildgebungsteil (220), der konfiguriert ist, basierend auf dem Detektionssignal ein Bild des Messobjekts zu bilden;
einen Bestimmungsteil (231), der konfiguriert ist, zu bestimmen, ob eine Intensität des Detektionssignals gleich oder mehr als ein vorbestimmter Schwellenwert ist;
**dadurch gekennzeichnet, dass** die Vorrichtung weiter umfasst:
eine Steuerung (210), die konfiguriert ist, mindestens eines von einer Scangeschwindigkeit und einer Scanposition des Signallichts durch den Scanner und eine Zeit zur Ansammlung von elektrischen Ladungen durch den Detektor zu steuern, um die Intensität des Detektionssignals des Interferenzlichts zu erhöhen, wenn bestimmt wird, dass die Intensität kleiner als der vorbestimmte Schwellenwert ist,
wobei der Bildgebungsteil (220) konfiguriert ist, basierend auf dem Detektionssignal mit der erhöhten Intensität ein Bild des Messobjekts zu bilden.

2. Optische Bildmessvorrichtung nach Anspruch 1, wobei:
die Steuerung (210) konfiguriert ist, den Scanner (141) zu steuern, mit dem Signallicht bei einer Scangeschwindigkeit zu scannen, die der erhöhten Ansammlungszeit entspricht.

3. Optische Bildmessvorrichtung nach Anspruch 1, wobei:
die Steuerung (210) konfiguriert ist, den Scanner (141) zu steuern, sodass er das Signallicht zu einer derartigen Anzahl an Abtastpunkten sequenziell ausstrahlt, die der erhöhten Ansammlungszeit entspricht.

4. Optische Bildmessvorrichtung nach Anspruch 3,
wobei die Steuerung (210) konfiguriert ist, die Breitbandlichtquelle (160) zu steuern, sodass sie eine Ausgabezeit des Lichts von der Breitbandlichtquelle (160) erhöht und dadurch den Ansammlungsbetrag erhöht.

5. Optische Bildmessvorrichtung nach Anspruch 4, wobei:
die Steuerung (210) konfiguriert ist, eine Scangeschwindigkeit zu erlangen, die der erhöhten Ansammlungszeit entspricht, und den Scanner (141) zu steuern, das Signallicht mit der erlangten Scangeschwindigkeit zu scannen.

6. Optische Bildmessvorrichtung nach Anspruch 4, wobei:
die Steuerung (210) konfiguriert ist, eine derartige Anzahl an Abtastpunkten zu erlangen, die der erhöhten Ausgabezeit entspricht, und den Scanner (141) zu steuern, das Signallicht zu der erlangten Anzahl an Abtastpunkten sequenziell auszustrahlen.

7. Optische Bildmessvorrichtung nach Anspruch 1,
wobei die Steuerung (210) konfiguriert ist, die Breitbandlichtquelle (160) zu steuern, sodass sie eine Ausgabeintensität des Lichts von der Breitbandlichtquelle (160) erhöht und dadurch den Ansammlungsbetrag erhöht.

8. Optische Bildmessvorrichtung nach Anspruch 7, wobei:
die Steuerung (210) konfiguriert ist, den Scanner (141) zu steuern, mit dem Signallicht bei einer Scangeschwindigkeit zu scannen, die der erhöhten Ausgabeintensität entspricht.

9. Optische Bildmessvorrichtung nach Anspruch 7, wobei:
der Interferenzlichterzeuger mit einem Scanner (141) ausgestattet ist, der konfiguriert ist, eine Position zu scannen, um das Signallicht auf das Messobjekt auszustrahlen; und
die Steuerung (210) konfiguriert ist, den Scanner (141) zu steuern, das Signallicht zu einer derartigen Anzahl an Abtastpunkten sequenziell auszustrahlen, die der erhöhten Ausgabeintensität entspricht.

10. Optische Bildmessvorrichtung nach Anspruch 1, wobei:
der Bildgebungsteil (220) mit einem Rechner (221) ausgestattet ist, der konfiguriert ist, Intensitäten von mehreren Frequenzkomponenten des Detektionssignals zu erlangen;
der Bestimmungsteil (231) konfiguriert ist, einen Mindestwert der Intensitäten der mehreren Frequenzkomponenten zu spezifizieren und zu bestimmen, ob der Mindestwert gleich oder mehr als ein voreingestellter Wert ist, und dadurch die Intensität des Detektionssignals zu bestimmen; und
die Steuerung (210) konfiguriert ist, wenn bestimmt wird, dass der Mindestwert kleiner als der voreingestellte Wert ist, die Intensität des Detektionssignals des Interferenzlichts zu erhöhen, sodass die Intensität einer Frequenzkomponente, die dem Mindestwert entspricht, gleich oder mehr als der voreingestellte Wert ist.

11. Optische Bildmessvorrichtung nach Anspruch 1, wobei:
der Bestimmungsteil (231) konfiguriert ist, einen Maximalwert von Pixelwerten von mehreren Pixeln des Bildes zu spezifizieren und zu bestimmen, ob der Maximalwert gleich oder mehr als ein voreingestellter Wert ist, und dadurch die Intensität des Detektionssignals zu bestimmen; und
wobei die Steuerung (210) konfiguriert ist, wenn bestimmt wird, dass der Maximalwert kleiner als der voreingestellte Wert ist, die Intensität des Detektionssignals des Interferenzlichts zu erhöhen, sodass der Pixelwert eines Pixels, der dem Maximalwert entspricht, gleich oder mehr wird als der voreingestellte Wert.

12. Optische Bildmessvorrichtung nach Anspruch 1, wobei:
der Bestimmungsteil (231) konfiguriert ist, Pixel mit Pixelwerten von gleich oder mehr als einem voreingestellten Wert aus mehreren Pixeln des Bildes zu spezifizieren und zu bestimmen, ob eine Anzahl der spezifizierten Pixel gleich oder mehr als eine vorbestimmte Anzahl ist, und dadurch die Intensität des Detektionssignals zu bestimmen; und
die Steuerung (210) konfiguriert ist, wenn bestimmt wird, dass die Anzahl kleiner als der voreingestellte Wert ist, die Intensität des Detektionssignals des Interferenzlichts zu erhöhen, sodass die Anzahl an Pixeln mit Pixelwerten gleich oder mehr als der voreingestellte Wert gleich oder mehr wird als die vorbestimmte Anzahl.

13. Optische Bildmessvorrichtung nach Anspruch 1, wobei:
der Bestimmungsteil (231) konfiguriert ist, zu bestimmen, ob eine neue Intensität des Detektionssignals des Interferenzlichts nach dem Steuern durch die Steuerung (210) gleich oder mehr als der vorbestimmte Schwellenwert ist; und
wobei die Steuerung (210) konfiguriert ist, wenn bestimmt wird, dass die neue Intensität kleiner als der vorbestimmte Schwellenwert ist, die Breitbandlichtquelle (160), den Detektor (184) und den Bildgebungsteil (220) zu steuern, zwei oder mehr Bilder zu bilden und die zwei oder mehr Bilder zu überlagern und dadurch ein neues Bild zu bilden.

14. Optische Bildmessvorrichtung nach Anspruch 1, wobei:
wobei der Bestimmungsteil (231) konfiguriert ist, zu bestimmen, ob eine neue Intensität des Detektionssignals nach dem Steuern durch die Steuerung (210) gleich oder mehr als der vorbestimmte Schwellenwert ist; und
die Steuerung (210) konfiguriert ist, wenn bestimmt wird, dass die neue Intensität kleiner als der vorbestimmte Schwellenwert ist, den Bildgebungsteil (220) zu steuern, eine vorbestimmte Bildverarbeitung zum Erhöhen einer Bildqualität an dem Bild auszuführen.

15. Optische Bildmessvorrichtung nach Anspruch 1, wobei:
der Bestimmungsteil (231) konfiguriert ist, zu bestimmen, ob eine neue Intensität des Detektionssignals des Interferenzlichts nach dem Steuern durch die Steuerung (210) gleich oder mehr als der vorbestimmte Schwellenwert ist; und
die Steuerung (210) konfiguriert ist, wenn bestimmt wird, dass die neue Intensität kleiner als der vorbestimmte Schwellenwert ist, den Bildgebungsteil (220) zu steuern, eine Amplitude des Detektionssignals zu erhöhen, um ein neues Detektionssignal zu erzeugen und ein Bild basierend auf dem neuen Detektionssignal zu bilden.

## Revendications

1. Dispositif de mesure d'image optique, comprenant :
une source de lumière à large bande (160) qui émet une lumière à faible cohérence ;
un générateur de lumière d'interférence configuré pour séparer la source de lumière à large bande en une lumière de signal et une lumière de référence, et superposer la lumière de signal propagée à travers un objet mesuré et la lumière de référence propagée à travers un objet de référence pour générer une lumière d'interférence ;
un scanner (141) configuré pour balayer une position pour rayonner la lumière de signal sur l'objet mesuré ;
un détecteur (184) configuré pour recevoir la lumière d'interférence pour convertir en charges électriques, et accumuler les charges électriques pour générer un signal de détection ;
une partie de formation d'image (220) configurée pour former une image de l'objet mesuré en se basant sur le signal de détection ;
une partie de détermination (231) configuré pour déterminer si une intensité du signal de détection est supérieure ou égale à un seuil prédéterminé ;
**caractérisé en ce que** le dispositif comprend en outre :
une commande (210) configurée pour commander au moins l'une d'une vitesse de balayage et d'une position de balayage de la lumière de signal par le scanner, et un temps d'accumulation de charges électriques par le détecteur de façon à augmenter l'intensité du signal de détection de la lumière d'interférence lorsqu'il est déterminé que l'intensité est inférieure au seuil prédéterminé,
dans lequel la partie de formation d'image (220) est configurée pour former une image de l'objet mesuré en se basant sur le signal de détection avec l'intensité augmentée.

2. Dispositif de mesure d'image optique selon la revendication 1, dans lequel :
la commande (210) est configurée pour commander le scanner (141) pour balayer avec la lumière de signal à une vitesse de balayage correspondant au temps d'accumulation augmenté.

3. Dispositif de mesure d'image optique selon la revendication 1, dans lequel :
la commande (210) est configurée pour commander le scanner (141) pour rayonner de façon séquentielle la lumière de signal sur un nombre de points de balayage qui correspond au temps d'accumulation augmenté.

4. Dispositif de mesure d'image optique selon la revendication 3,
dans lequel la commande (210) est configurée pour commander la source de lumière à large bande (160) pour augmenter un temps d'émission de la lumière depuis la source de lumière à large bande (160), augmentant ainsi la quantité d'accumulation.

5. Dispositif de mesure d'image optique selon la revendication 4, dans lequel :
la commande (210) est configurée pour obtenir une vitesse de balayage correspondant au temps d'accumulation augmenté, et pour commander le scanner (141) pour balayer la lumière de signal avec la vitesse de balayage obtenue.

6. Dispositif de mesure d'image optique selon la revendication 4, dans lequel :
la commande (210) est configurée pour obtenir un nombre de points de balayage qui correspond au temps d'émission augmenté, et pour commander le scanner (141) pour rayonner la lumière de signal de façon séquentielle sur le nombre obtenu de points de balayage.

7. Dispositif de mesure d'image optique selon la revendication 1,
dans lequel la commande (210) est configurée pour commander la source de lumière à large bande (160) pour augmenter une intensité d'émission de la lumière depuis la source de lumière à large bande (160), augmentant ainsi la quantité d'accumulation.

8. Dispositif de mesure d'image optique selon la revendication 7, dans lequel :
la commande (210) est configurée pour commander le scanner (141) pour balayer avec la lumière de signal à une vitesse de balayage correspondant à l'intensité d'émission augmentée.

9. Dispositif de mesure d'image optique selon la revendication 7, dans lequel :
le générateur de lumière d'interférence est doté d'un scanner (141) configuré pour balayer une position pour rayonner la lumière de signal sur l'objet mesuré ; et
la commande (210) est configurée pour commander le scanner (141) pour rayonner la lumière de signal de façon séquentielle sur un nombre de points de balayage qui correspond à l'intensité d'émission augmentée.

10. Dispositif de mesure d'image optique selon la revendication 1, dans lequel :
la partie de formation d'image (220) est dotée d'un calculateur (221) configuré pour obtenir des intensités d'une pluralité de composantes de fréquence du signal de détection ;
la partie de détermination (231) est configurée pour spécifier une valeur minimale des intensités de la pluralité de composantes de fréquence et déterminer si la valeur minimale est supérieure ou égale à une valeur prédéterminée, déterminant ainsi l'intensité du signal de détection ; et
la commande (210) est configurée pour, lorsqu'il est déterminé que la valeur minimale est inférieure à la valeur prédéterminée, augmenter l'intensité du signal de détection de la lumière d'interférence de façon à ce que l'intensité d'un composant de fréquence correspondant à la valeur minimale devienne supérieure ou égale à la valeur prédéterminée.

11. Dispositif de mesure d'image optique selon la revendication 1, dans lequel :
la partie de détermination (231) est configurée pour spécifier une valeur maximale de valeurs de pixels d'une pluralité de pixels de l'image et déterminer si la valeur maximale est supérieure ou égale à une valeur prédéterminée, déterminant ainsi l'intensité du signal de détection ; et
la commande (210) est configurée pour, lorsqu'il est déterminé que la valeur maximale est inférieure à la valeur prédéterminée, augmenter l'intensité du signal de détection de la lumière d'interférence de façon à ce que la valeur de pixel d'un pixel correspondant à la valeur maximale devienne supérieure ou égale à la valeur prédéterminée.

12. Dispositif de mesure d'image optique selon la revendication 1, dans lequel :
la partie de détermination (231) est configurée pour spécifier des pixels ayant des valeurs de pixels supérieures ou égales à une valeur prédéterminée parmi une pluralité de pixels de l'image, et déterminer si un nombre des pixels spécifiés est supérieur ou égal à un nombre prédéterminé, déterminant ainsi l'intensité du signal de détection ; et
la commande (120) est configurée pour, lorsqu'il est déterminé que le nombre est inférieur à la valeur prédéterminée, augmenter l'intensité du signal de détection de la lumière d'interférence de façon à ce que le nombre de pixels ayant des valeurs de pixel supérieures ou égales à la valeur prédéterminée devienne supérieur ou égal au nombre prédéterminé.

13. Dispositif de mesure d'image optique selon la revendication 1, dans lequel :
la partie de détermination (231) est configurée pour déterminer si une nouvelle intensité du signal de détection de la lumière d'interférence après la commande par la commande (210) est supérieure ou égale au seuil prédéterminé ; et
la commande (210) est configurée pour, lorsqu'il est déterminé que la nouvelle intensité est inférieure au seuil prédéterminé, commander la source de lumière à large bande (160), le détecteur (184) et la partie de formation d'image (220) pour former deux ou plusieurs images et superposer les deux ou plusieurs images, formant ainsi une nouvelle image.

14. Dispositif de mesure d'image optique selon la revendication 1, dans lequel :
la partie de détermination (231) est configurée pour déterminer si une nouvelle intensité du signal de détection après la commande par la commande (210) est supérieure ou égale au seuil prédéterminé ; et
la commande (210) est configurée pour, lorsqu'il est déterminé que la nouvelle intensité est inférieure au seuil prédéterminé, commander la partie de formation d'image (220) pour exécuter un traitement d'image prédéterminé pour augmenter une qualité d'image sur l'image.

15. Dispositif de mesure d'image optique selon la revendication 1, dans lequel :
la partie de détermination (231) est configurée pour déterminer si une nouvelle intensité du signal de détection de la lumière d'interférence après la commande par la commande (210) est supérieure ou égale au seuil prédéterminé ; et
la commande (210) est configurée pour, lorsqu'il est déterminé que la nouvelle intensité est inférieure au seuil prédéterminé, commander la partie de formation d'image (220) pour augmenter une amplitude du signal de détection pour générer un nouveau signal de détection et former une image en se basant sur le nouveau signal de détection.
